(19) Europäisches Patentamt
European Patent Office
Office européen des brevets

(11) Numéro de publication : **0 668 270 A2**

(12) # DEMANDE DE BREVET EUROPEEN

(21) Numéro de dépôt : **95420037.4**

(22) Date de dépôt : **16.02.95**

(51) Int. Cl.[6] : **C07D 233/86,** C07D 401/04, C07D 235/02, C07D 277/36, C07D 263/42, C07C 331/28, C07D 233/76, C07D 233/78, C07D 233/84, A01N 43/50

(30) Priorité : **17.02.94 FR 9402135**

(43) Date de publication de la demande : **23.08.95 Bulletin 95/34**

(84) Etats contractants désignés : **AT BE CH DE DK ES FR GB GR IE IT LI LU NL PT SE**

(71) Demandeur : **RHONE-POULENC AGROCHIMIE**
**14-20, rue Pierre Baizet**
**F-69009 Lyon (FR)**

(72) Inventeur : **Bascou, Jean-Philippe**
**3, rue Gabriel Chevallier**
**F-69009 Lyon (FR)**
Inventeur : **Gadras, Alain**
**208 Rue de St Cyr**
**F-69009 Lyon (FR)**
Inventeur : **Perez, Joseph**
**19 Allée D Rue Ernest Fabrègue**
**F-69009 Lyon (FR)**
Inventeur : **Emeric, Gilbert**
**97 Chemin des Tuileries**
**F-69570 Dardilly (FR)**
Inventeur : **Lacroix, Guy**
**332F Rue du Doyen Chapas**
**F-69009 Lyon (FR)**
Inventeur : **Veyrat, Christine**
**21 Rue Nervieux**
**F-69450 St Cyr au Mont d'Or (FR)**

(54) **Dérivés de 2-imidazoline-5-ones fongicides.**

(57) L'invention concerne des dérivés de 2-imidazoline-5-ones, de formule générale (I) :

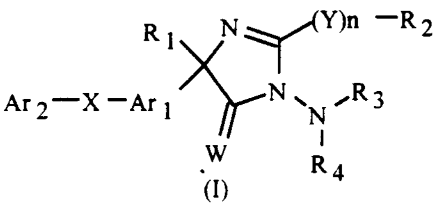

(I)

dans laquelle :

— $R_1$ est H, ou un groupe vinyle ou allyle, ou un radical alkyle ou haloalkyle,

— n = 0 ou 1 ; Y est O ou S ;

— $R_2$ est un groupe alkyle ou haloalkyle, ou le groupe cyclopropyle ;

— $R_3$ est un radical aryle ou hétéroaryle, éventuellement substitué, comprenant notamment phényle, naphtyle,

— $R_4$ est un atome d'hydrogène et divers radicaux dont un radical formyle ou aroyle, un radical acyle de 2 à 6 atomes de carbone,

— $R_5$ est notamment H ou le groupe hydroxy, mercapto, nitro, cyano, thiocyanato ou azido ; ou un radical alkyle ou cycloalkyle éventuellement subsitué,

— W est O, S ou SO ;

— $Ar_1$ est un radical divalent, éventuellement substitué, qui dérive d'un radical aryle ou hétéroaryle comprenant, notamment, les radicaux phényle, naphtyle, thiényle,

— $Ar_2$ est un système mono ou bicyclique de 5 à 10 atomes, éventuellement substitué, qui est aromatique, saturé ou insaturé, et qui est soit un système carbocyclique, soit un système hétérocyclique contenant de 1 à 4 hétéroatomes choisis parmi O, S, ou N,

— X est notamment -C(O)NH-, -NHC(O)NH-, $-SO_2NH-$, $-CH_2O-$, $-CH_2NH-$, - $CH_2$-.

L'invention concerne également la préparation de ces composés et leur utilisation comme fongicide à spectre large.

EP 0 668 270 A2

La présente invention concerne de nouveaux composés avec un groupe 2-imidazoline-5-one à usage phytosanitaire. Elle concerne également les procédés de préparation desdits composés et les produits éventuellement utilisables à titre d'intermédiaires dans les procédés de préparation. Elle concerne enfin les compositions fongicides à base de ces composés et les procédés pour lutter contre les maladies fongiques des cultures utilisant ces composés.

On connaît des 2-imidazoline-5-ones à usage phytosanitaire par la demande de brevet européen EP 0 551048 et par les demandes internationales WO 93/24467 et WO 94/01410.

Un but de la présente invention est de proposer de nouveaux composés comportant un groupe 2-imidazoline-5-one présentant des propriétés améliorées dans le traitement des maladies fongiques des cultures.

Un autre but de la présente invention est de proposer des composés présentant un spectre d'utilisation également amélioré dans le domaine des maladies fongiques, notamment pour le traitement des maladies fongiques du riz et pour le traitement de la rouille brune du blé.

Il a maintenant été trouvé que ces buts pouvaient être atteints, en totalité ou en partie, au moyen des produits de l'invention, décrits ci-après.

L'invention a pour objet en premier lieu des composés qui sont des dérivés de 2-imidazoline-5-ones de formule générale (I) :

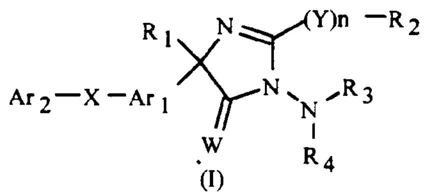

(I)

dans laquelle :

- $R_1$ représente un atome d'hydrogène, ou un groupe vinyle ou allyle, ou un radical alkyle ou haloalkyle, chacun de 1 à 3 atomes de carbone ;

  ou encore, $R_1$ et $Ar_2$-X-$Ar_1$ peuvent en outre former, avec le carbone auquel ils sont liés sur le cycle imidazolinone, le motif suivant :

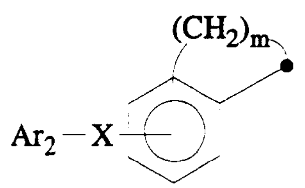

  dans lequel le point en caractère gras représente le carbone du cycle imidazolinone de la formule (I) auquel sont liés les radicaux $R_1$ et $Ar_2$-X-$Ar_1$, et m égale 2, 3 ou 4 ;
- n = 0 ou 1 ;
- Y représente l'atome d'oxygène ou de soufre ;
- $R_2$ représente :
  - l'atome d'hydrogène quand n égale 0 ou
  - un groupe alkyle ou haloalkyle, chacun de 1 à 3 atomes de carbone ou le groupe cyclopropyle ;
- $R_3$ représente un radical aryle ou hétéroaryle comprenant phényle, naphtyle, pyridyle, pyrimidyle, pyridazinyle, pyrazinyle, thiazolyle, benzothiényle, benzofuryle, quinolinyle, isoquinolinyle, benzothiazolyle ou méthylène dioxyphényle, chacun de ces radicaux étant éventuellement substitué par 1 à 7 groupements, de préférence de 1 à 3 groupements, choisis parmi les significations de $R_5$ défini ci-après ;
- $R_4$ représente un atome d'hydrogène, un radical formyle ou aroyle, un radical acyle de 2 à 6 atomes de carbone, un radical alkoxycarbonyle de 2 à 6 atomes de carbone, un radical aryloxycarbonyle ou arylsulfonyle, un radical alkylsulfonyle de 1 à 6 atomes de carbone, ou un radical alkyloxalyle ou alkoxyoxalyle de 3 à 6 atomes de carbone ;
- $R_5$ représente:
  - un atome d'halogène ou le groupe hydroxy, mercapto, nitro, $SF_5$, cyano, thiocyanato ou azido; ou
  - un radical alkyle, haloalkyle, cyanoalkyle, alkoxy, haloalkoxy, cyanoalkoxy, alkylthio, haloalkylthio, cyanoalkylthio, alkylsulfmyle, alkylsulfonyle, haloalkylsulfinyle ou haloalkylsulfonyle, chacun de 1 à 6 atomes de carbone ; ou

- un radical cycloalkyle, halocycloalkyle, alcényle, alcynyle, alcényloxy, alcynyloxy, alcénylthio, alcynylthio, chacun de 3 à 6 atomes de carbone ; ou
- un radical amino éventuellement mono ou disubstitué par un radical alkyle ou acyle de 1 à 6 atomes de carbone ou alkoxycarbonyle de 2 à 6 atomes de carbone ;ou
- un groupe alkoxycarbonyle de 2 à 7 atomes de carbone ; ou
- un groupe N-alkylcarbamoyle de 2 à 7 atomes de carbone ; ou
- un groupe N,N-dialkylcarbamoyle de 3 à 13 atomes de carbone ;

- W représente O, S ou SO ;
- $Ar_1$ est un radical divalent qui dérive d'un radical aryle ou hétéroaryle comprenant les radicaux phényle, naphtyle, thiényle, furyle, pyrrolyle, pyridyle, benzothiényle, benzofuryle, indolyle, quinolinyle, isoquinolinyle, ou méthylènedioxyphényle, chacun de ces radicaux étant éventuellement substitué par 1 à 6 groupements, de préférence par 1 à 3 groupements, choisis parmi les significations de $R_5$ ;
- $Ar_2$ représente un système mono ou bicyclique de 5 à 10 atomes, qui est aromatique, saturé ou insaturé, et qui est soit un système carbocyclique, soit un système hétérocyclique contenant de 1 à 4 hétéroatomes choisis parmi O, S, ou N, et qui comprend les radicaux phényle, naphtyle, dihydronaphtyle, tétrahydronaphtyle, thiényle, furyle, pyrrolyle, pyridyle, benzothiényle, benzofuryle, indanyle, indolyle, quinolinyle, isoquinolinyle, méthylènedioxyphényle, imidazolyle, pyrazolyle, triazolyle, oxazolyle, isoxazolyle, thiazolyle, isothiazolyle, oxadiazolyle, thiadiazolyle, benzimidazolyle, indazolyle, benzoxazolyle, benzisoxazolyle, benzothiazolyle, benzisothiazolyle, pyrimidyle, pyrazinyle, pyridazinyle, triazinyle, naphtyridyle, quinoxazolyle, quinazolyle, cinnolyle ou phtalazinyle, chacun de ces radicaux étant éventuellement substitué par par 1 à 7 groupements, de préférence par 1 à 3 groupements, choisis parmi les significations de $R_5$ ;
- X représente un enchaînement de formule générale :

$$-(R'')_j-A-(R')_k-$$

  dans lequel :
  - j et k, identiques ou différents, sont égaux à 0 ou 1 ;
  - R"et R', identiques ou différents, représentent une chaîne hydrocarbonée, saturée ou insaturée, qui contient de 1 à 6 atomes de carbone, éventuellement substituée par 1 à 12 groupements, de préférence par 1 à 3 groupements, choisis parmi $R_7$ défini ci-après ; et dans laquelle un ou plusieurs atomes de carbone peuvent être remplacés par O, S, $N(R_5)$, $R_5$ étant défini ci-après ;
  - A représente O, S, $N(R_5)$, SO, $SO_2$, CO, CS, , $Si(R_8)(R'_8)$, $N_2$, $C(R_{10})(R'_{10})$, ou -C≡C- ou

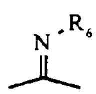 ou 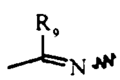 ou 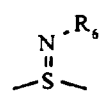 ou 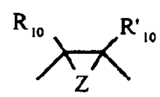

ou

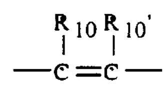

dans lequel Z représente O,S, $C(R_{11})(R'_{11})$; $R_8$ à $R_{11}$ étant définis ci-après ;

- $R_5$ représente :
  - un atome d'hydrogène ou un groupe cyano, hydroxy, amino, formyle, morpholino, pipéridino, pyrrolidino, pipérazino, ou
  - un radical alkyle, haloalkyle, cyanoalkyle, alkoxy, haloalkoxy, cyanoalkoxy, alkylsulfonyle, haloalkylsulfonyle, cyanoalkylsulfonyle, chacun comprenant de 1 à 6 atomes de carbone, ou
  - un radical monoalkylamino ou dialkylamino, ou
  - un radical cycloalkyle, halocycloalkyle, alcényle, alcynyle, alcényloxy, alcynyloxy, chacun de 3 à 6 atomes de carbone ; ou
  - un radical acyle ou alkoxycarbonyle, chacun de 2 à 6 atomes de carbone ; ou
  - un radical carbamoyle (éventuellement substitué par un ou deux radicaux alkyle) de 1 à 7 atomes de carbone ou sulfamoyle de 1 à 6 atomes de carbone ; ou
  - un radical aroyle ou arylsulfonyle ; ou
  - un radical alkyloxalyle ou alkoxyoxalyle, chacun de 3 à 8 atomes de carbone ; ou
  - un radical oxamyle (éventuellement substitué par un ou deux radicaux alkyle) de 2 à 8 atomes de

carbone

- $R_7$ représente un atome d'halogène, un groupe cyano, thiocyanato, hydroxycarbonyl, amino (éventuellement substitué), hydroxyle, oxo, alkoxy, alkoxycarbonyl, haloalkoxy, alkoxyalkoxy, thiol, alkylthio, haloalkylthio, alkoxyalkylthio, acyloxy, alkyle, haloalkyle, alkoxyalkyle, alkylidène, aroyloxy, hétéroaroyloxy, arylacyloxy, cycloalkylcarbonyloxy, acylthio, aroylthio, hétéroaroylthio, arylacylthio, cycloalkylcarbonylthio, carbamoyloxy (éventuellement substitué), carbamoylthio (éventuellement substitué), thiocarbamoyloxy (éventuellement substitué), dithiocarbamoyle (éventuellement substitué), acylamino, cycloalkylcarbonylamino, aroylamino, uréïdo (éventuellement substitué), thiouréïdo, alkoxycarbonylamino, aryloxycarbonylamino, alkylsulfonylamino, arylsulfonylamino, aminosulfonylamino (éventuellement substitué), étant entendu que:
  - par éventuellement substitué on entend des radicaux éventuellement substitués par un ou deux radicaux alkyles,
  - chaque radical hydrocarboné aliphatique a de 1 à 4 atomes de carbone, et
  - le radical cycloalkyle contient de 3 à 7 atomes de carbone ;
- $R_8$ et $R'_8$, identiques ou différents, représentent :
  - un radical alkyle de 1 à 6 atomes de carbone ; ou
  - un radical cycloalkyle de 3 à 7 atomes de carbone ; ou
  - un radical alcényle, alcynyle de 2 à 6 atomes de carbone ; ou
  - un radical arylalkyle, de préférence benzyle, ou un radical aryle éventuellement substitué, de préférence phényle éventuellement substitué par 1 à 5 groupements, de préférence par 1 à 3 groupements, choisis parmi les significations de $R_8$ ;
- $R_9$ représente :
  - l'atome d'hydrogène, ou un groupe morpholino, pipéridino, pyrrolidino, pipérazino ; ou
  - un radical alkyle, haloalkyle, cyanoalkyle, alkoxy, haloalkoxy, cyanoalkoxy, alkylthio, haloalkylthio, cyanoalkylthio, chacun de 1 à 6 atomes de carbone ou dialkylamino contenant de 2 à 6 atomes de carbone ;
- $R_{10}$ et $R'_{10}$, identiques ou différents, représentent l'atome d'hydrogène ou un groupe alkoxy contenant 1 à 6 atomes de carbone ou $R_7$ ;
- $R_{11}$ et $R'_{11}$, identiques ou différents, représentent l'atome d'hydrogène ou un halogène ou un groupe alkyle de 1 à 3 atomes de carbone ;

ainsi que les formes salifiées, et les énantiomères et stéréoisomères de ces composés, notamment les 2 énantiomères de chacun de ces composés qui correspondent au carbone asymétrique du cycle imidazolinone porteur de $R_1$ ;

et à l'exception des composés pour lesquels :

- lorsque $(Y)_n$-$R_2$ représente un méthylthio, un méthyle ou un éthyle, $Ar_2$-X-$Ar_1$ représente un groupe phénoxyphényle, éventuellement substitué;
- lorsque $(Y)_n$-$R_2$ représente un méthoxy, lorsque $R_1$ est un méthyle, lorsque $R_3$ représente un radical phényle ou pyridyle, éventuellement substitué par un atome de fluor ou un groupe méthyle, lorsque $R_4$ est un hydrogène, et lorsque W représente un atome d'oxygène, alors $Ar_2$-X-$Ar_1$ représente soit un groupe 4-phénoxyphényle, éventuellement substitué sur sa partie phénoxy par 1 ou 2 atomes de fluor, soit le groupe 3-phénoxyphényle ;

et à l'exception du composé de formule :

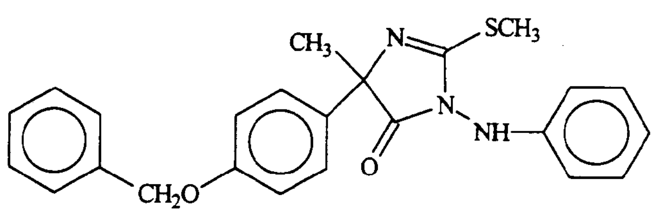

Dans le présent texte, les termes génériques alkyle, acyle, et aroyle, ont les significations suivantes :

- alkyle signifie un radical aliphatique linéaire ou ramifié ;
- acyle signifie :
  - un groupe alkylcarbonyl ou haloalkylcarbonyl ; ou
  - un groupe alkoxyalkylcarbonyl ou alkylthioalkylcarbonyl ; ou
  - un groupe alcénylcarbonyl ; ou
  - un groupe alcynylcarbonyl ; ou

- un groupe cycloalkylcarbonyl ou halocycloalkylcarbonyl ;
- aroyle signifie un groupe arylcarbonyle, le groupe aryle ayant les mêmes significations que celles indiquées comme préférées pour $Ar_2$, et étant éventuellement substitué par $R_5$.

On préfère les composés de formule (I) dans laquelle :

- $Ar_1$ représente un radical divalent dérivé d'un phényle, naphtyle, thiényle, pyridyle, benzothiényle, quinolinyle, chacun de ces radicaux étant éventuellement substitué par 1 à 3 groupements, choisis parmi les significations de $R_5$ ;
- $Ar_2$ représente un radical phényle, naphtyle, thiényle, pyridyle, benzothiényle, thiazolyle, benzothiazolyle, pyrimidyle, chacun de ces radicaux étant éventuellement substitué par 1 à 3 groupements, choisis parmi les significations de $R_5$.

Sont particulièrement préférés les composés de formule (I) dans laquelle :

- $Ar_1$ représente un radical divalent dérivé d'un phényle, thiényle, pyridyle, éventuellement substitué par 1 à 3 groupements choisis parmi un atome d'halogène, un radical alkyle ou haloalkyle ;
- $Ar_2$ représente un radical phényle, naphtyle, pyridyle, benzothiazolyle, éventuellement substitué par 1 à 3 groupements choisis parmi un atome d'halogène, un radical alkyle ou haloalkyle ;
- X représente l'enchaînement de formule générale indiquée précédemment dans laquelle :
  - R" et R', identiques ou différents, représentent une chaîne carbonée de 1 à 3 atomes de carbone, de préférence un groupe méthylène, dans laquelle un atome de carbone est éventuellement remplacé par O ou NH, et qui est éventuellement substituée par 1 à 3 groupements choisis parmi un atome d'halogène, un groupe hydroxyle, oxo ou un radical $C_1$-$C_3$ alkyle ;
  - A est O, S, SO, $SO_2$, CO, $C(R_{10})(R'_{10})$,

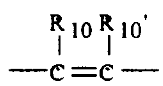 ou 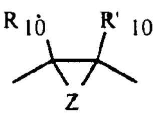

dans lesquels :

- $R_{10}$ et $R'_{10}$, identiques ou différents, sont un atome d'hydrogène, d'halogène, un radical hydroxyle, $C_1$-$C_3$ alkyle ou $C_1$-$C_3$ alkoxy ;
- Z représente O ou $C(R_{11})(R'_{11})$, où $R_{11}$ et $R'_{11}$, identiques ou différents, sont un atome d'hydrogène ou de chlore.

Enfin, des composés tout particulièrement avantageux sont obtenus en choisissant des dérivés de formule I dans laquelle simultanément :

- $R_1$ est un radical méthyle ;
- $R_2$ est un radical méthyle ou éthyle ;
- $R_3$ représente un radical phényle ou pyridyle ;
- $R_4$ est un atome d'hydrogène ou un radical formyle ;
- $R_5$ est un atome d'halogène ou un radical $C_1$-$C_3$ alkyle ou $C_1$-$C_3$ haloalkyle ; et
- W est un atome d'oxygène.

Les composés de formule (I) sont préparés selon les procédés explicités ci-après. Tous les groupes apparaissant dans les formules chimiques qui suivent, et déjà définis dans la formule générale (I), conservent la même signification, pour autant qu'il n'en soit pas précisé autrement. Dans la description générale des procédés de préparations des composés de formule (I), les conditions données sont celles qui sont généralement utilisées : il convient de préciser que des conditions autres que celles spécifiées peuvent être utilisées, dépendant par exemple des réactifs particuliers utilisés.

Les structures de tous les produits qui illustrent ces procédés ont été établies par au moins 1 des techniques spectrales suivantes : spectrométrie RMN du proton, spectrométrie RMN du carbone 13, spectrométrie Infra-Rouge et spectrométrie de masse.

Dans les tableaux qui rassemblent ces produits, en indiquant les significations des groupes d'une formule générale, les radicaux méthyle, éthyle, propyle, pyridyle, pyrimidinyle, acétyle, phényle et benzyle sont respectivement représentés par Me, Et, Pr, Py, Pyrim, Ac, Ph et Bn, et PF signifie point de fusion (PF).

Les composés de formule (I), pour lesquels $R_4$ est différent de l'atome d'hydrogène, sont préparés à partir des composés de formule Ia par réaction avec un composé de formule $R_4L'$, en présence d'une base et en milieu solvant, selon le schéma réactionnel suivant :

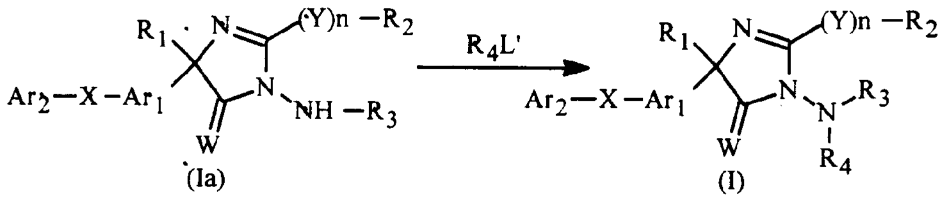


(Ia) (I)

Dans ce schéma :

- $R_4$ représente le radical formyle, un groupe acyle de 2 à 6 atomes de carbone, aroyle, alkoxycarbonyle de 2 à 6 atomes de carbone,
  aryloxycarbonyle, alkylsulfonyle, arylsulfonyle, ou un radical alkyloxalyle ou alkoxyoxalyle de 3 à 6 atomes de carbone ;
- L' représente :
  un atome d'halogène, de préférence chlore, brome ou iode, le groupe sulfate,
  un radical aryloxy ou arylthio, éventuellement substitué, de préférence phénoxy,
  un radical alkoxy ou dialkylamino,
  un groupe $R_4O$ lorsque $R_4$ est un groupe acyle ou arylsulfonyl ou alkylsulfonyl.

Comme base, on utilise une base forte telle qu'un hydrure ou hydroxyde alcalin ou alcalinoterreux, un alcoolate ou une amine tertiaire. La réaction est pratiquée à une température comprise entre -30°C et + 50°C. Le solvant est choisi parmi les éthers cycliques ou non cycliques, le diméthylformamide, le diméthylsulfoxyde, l'acétonitrile ou un solvant aromatique.

La préparation du composé de formule Ia est décrite selon 2 modes de réalisation.

On décrit tout d'abord un premier mode de préparation du composé de formule Ia, selon lequel on construit le cycle imidazolinone à partir d'intermédiaires contenant le groupement $Ar_2$-X-$Ar_1$. Ce premier mode de préparation des composés selon l'invention correspond aux procédés A, B, C, D décrits ci-après.

Préparation des composés de formule Ia dans laquelle n = 1 et Y = S :

**Procédé A :**

Les composés de formule (Ia) pour lesquels n=1 et Y=S et W = S ou O, autrement dit les composés de formule II, sont préparés selon un des procédés décrits dans la demande de brevet européen EP 0 551 048, notamment par S-alkylation des 2-thiohydantoïnes de formule (III), dans laquelle W représente S ou O, en présence d'une base et dans un solvant, selon le schéma réactionnel suivant :

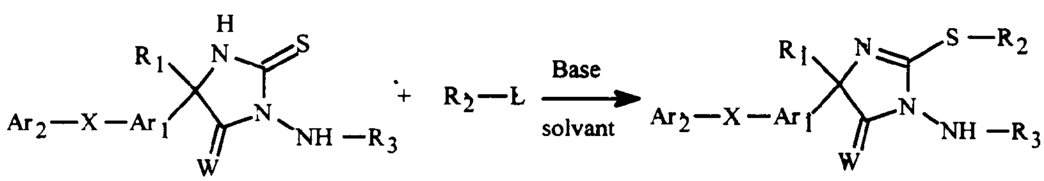


(III) (II)

dans lequel, L représente un groupement choisi parmi un atome d'halogène, (de préférence chlore, brome, ou iode ), ou le groupe sulfate d'alkyle, alkylsulfonyloxy, ou arylsulfonyloxy. Comme base on utilise une base forte telle qu'un alcoolate alcalin ou alcalino-terreux (de préférence du tertiobutylate de potassium), un hydroxyde alcalin ou alcalino-terreux, un carbonate alcalin ou alcalino-terreux ou une amine tertiaire. Comme solvant on utilise un éther cyclique ou non cyclique, un ester d'alkyle, l'acétonitrile, un alcool de 1 à 4 atomes de carbone, un solvant chloré, un solvant aromatique. On utilise de préférence comme solvant le tétrahydrofurane (THF). La réaction est mise en oeuvre à une température comprise entre -5°C et +80°C.

L'exemple suivant illustre ce procédé.

Exemple CF1 :

Préparation de la (4-R,S)-4-méthyl-2-méthylthio-1-phénylamino-4-(4-(2-phényléthyl)phényl)-2-imidazoline-5-thione (Composé n° 14).

A 5 g (0,012 mol) de (4-R,S)-4-méthyl-1-phénylamino-4-(4-(2-phényléthyl)phényl) imidazolidine-2,5-dithione dissous dans 150 ml de THF sont ajoutés à 0°C 1,36g (0,012 mol) de tertiobutylate de potassium. Après 15 minutes d'agitation à cette température, 1,5 ml (0,024 mol) d'iodure de méthyle sont ajoutés. L'agitation est poursuivie à 0°C pendant 30 minutes, puis après avoir laissé la température remonter à l' ambiante, le milieu est hydrolysé puis extrait au chlorure de méthylène. Après lavage des extraits organiques avec une solution saturée de chlorure de sodium, décantation, séchage et évaporation des solvants, une huile marron est obtenue. On cristallise cette huile dans un mélange d'éther diisopropylique et de pentane. Après filtration, on récupére 2,7 g (0,006 mol) de (4-R,S)-4-méthyl-2-méthylthio-1-phénylamino-4-(4-(2-phényléthyl)phényl)-2-imidazoline-5-thione sous la forme de poudre marron fondant à 118°C avec un rendement de 49%.

On décrit à présent la préparation du composé de formule III selon que W signifie S (composé de formule IIIa) ou O (composé de formule IIIb).

Préparation du composé de formule IIIa :

Les dithiohydantoïnes de formule (IIIa) sont obtenues à partir d'un composé de formule (IV), selon un procédé analogue à celui décrit par T.Yamamoto et Coll.dans J. Chem. Soc. Perkin Trans I (1990) Page 3003 à 3009, c'est-à-dire selon la réaction :

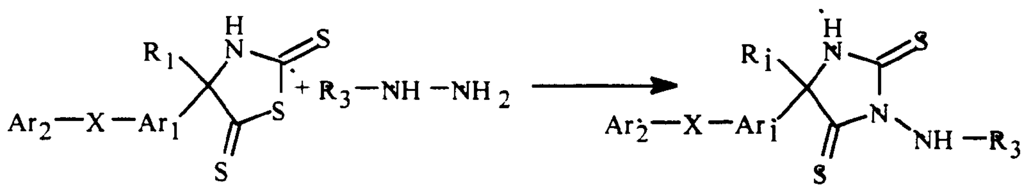

(IV) (IIIa)

L'exemple suivant illustre la préparation d'un composé de formule (IIIa) :

Exemple CI1 :

Préparation de (4-R,S)-4-méthyl-1-phénylamino-4-(4-(2-phényléthyl)phényl) imidazolidine-2,5-dithione, de formule développée :

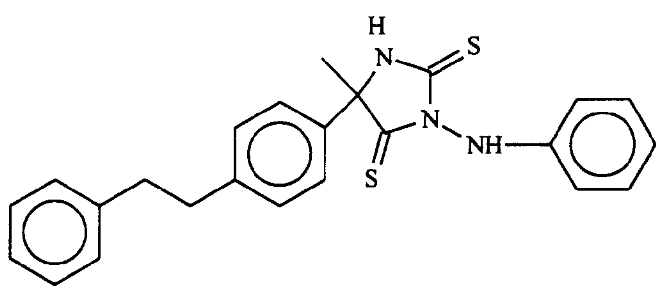

A 22,6 g (0,066 mol) de (4-R,S)-4-méthyl-4-(4-(2-phényléthyl)phényl) thiazolidine 2,5-dithione dans 100ml de toluène, on ajoute 6,7ml (0,066 mol) de phénylhydrazine. Le milieu réactionnel est chauffé progressivement à 60°C puis à reflux jusqu'à ce que le dégagement gazeux cesse. Après refroidissement du milieu, on procède à une hydrolyse, puis à une extraction à l'acétate d'éthyle. Après décantation, séchage de la phase organique et évaporation des solvants, une huile noire est obtenue. Après chromatographie sur colonne de silice (Acétate d'éthyle / heptane 50 / 50), on récupère 11,2 g (0,027 mol) de (4-R,S)-4-méthyl-1-phénylamino-4-(4-(2-phényléthyl)phényl)imidazolidine-2,5-dithione sous forme de poudre marron fondant à 171°C avec un rendement de 41%.

Les thiazolidine 2,5-dithiones de formule (IV) sont obtenues à partir d'un isothiocyanate de formule (V), selon un procédé analogue à celui décrit par T.Yamamoto et Coll. dans J.Chem.Soc.Perkin Trans I (1990) Page

2459 à 2463, selon la réaction :

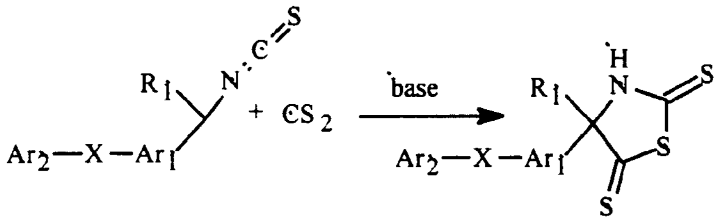


(V) (IV)

L'exemple suivant illustre la préparation d'un composé de formule (IV) :

<u>Exemple CI2</u> :

Préparation de la (4-R,S)-4-méthyl-4-(4-(2-phényléthyl)phényl) thiazolidine 2,5-dithione, de formule développée :

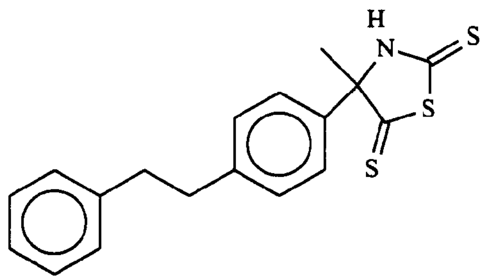

A 9,5 g (0,084 mol) de tertiobutylate de potassium placés dans 150ml de THF à -70°C, est ajoutée lentement une solution de 7ml (0,11 mol) de sulfure de carbone et de 20,4 g (0,076 mol) de 1-[4-(2-phényléthyl)phényl]éthylisothiocyanate dans 50 ml de THF. Après 15 minutes d'agitation à -70°C on laisse la température remonter à l'ambiante. Après hydrolyse du milieu réactionnel avec une solution aqueuse d'acide acétique, extraction à l'acétate d'éthyle, décantation, séchage des extraits organiques et évaporation des solvants,on récupère 22,6g (0,065 mol) de (4-R,S)-4-méthyl-4-(4-(2-phényléthyl)phényl)thiazolidine-2,5-dithione sous forme d'huile avec un rendement brut de 86%.

L' isothiocyanate de formule (V) est préparé à partir d'une amine de formule (VI) dans laquelle les substituants ont la même signification que dans (V), selon un des procédés cités dans Sulfur Reports (1989) Volume 8 (5) pages 327-375, soit selon la réaction :

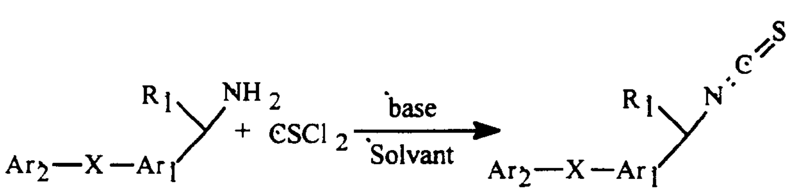


(VI) (V)

L'exemple suivant illustre la préparation d'un composé de formule (V) :

<u>Exemple CI3</u> :

Préparation du 1-[4-(2-phényléthyl)phényl]éthylisothiocyanate, de formule développée :

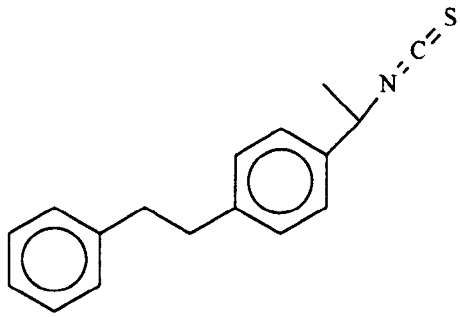

A une suspension de 20 g (0,076 mol) de chlorhydrate de 1-(4-(2-phényléthyl)phényl)éthylamine dans 200 ml de toluène à 0°C est ajouté 1 équivalent d'hydrogénocarbonate de sodium en solution aqueuse. Après avoir ramené la température du milieu réactionnel à température ambiante, 2 équivalents d'hydrogènocarbonate de sodium en solution aqueuse sont ajoutés. 6,1ml (0,076 mol) de thiophosgène en solution dans 10 ml de toluène sont alors ajoutés lentement. A la fin de la coulée, l'agitation est poursuivie pendant 15 minutes. Après décantation, les extraits organiques sont lavés à l'eau, séchés puis évaporés. On obtient ainsi 20,5g (0,076 mol) de 1-[4-(2-phényléthyl)phényl]éthylisothiocyanate sous forme d'huile avec un rendement de 100%.

Préparation du composé de formule IIIb :

Les 2-thiohydantoïnes de formule (IIIb), composés de formule (III) dans laquelle W est l'atome d'oxygène, sont obtenues selon le procédé décrit dans la demande de brevet européen EP 0 551 048, par une réaction de cyclisation des isothiocyanates de formule (VII) avec les hydrazines de formule $R_3$-NH-$NH_2$ selon le schéma réactionnel suivant :

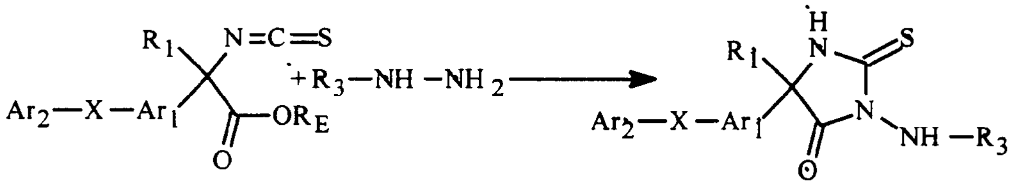

(VII) (IIIb)

dans lequel $R_E$ représente un radical $C_1$-$C_3$ alkyle.
La cyclisation peut être mise en oeuvre selon 2 modes de réalisation.
Selon le premier, on chauffe le mélange des réactifs à une température comprise entre 110°C et 180°C, dans un solvant aromatique tel que le toluène, le xylène, les chlorobenzènes ou le nitrobenzène.
Selon le second mode de réalisation de la cyclisation, on procède en présence d'une base forte telle qu'un alcoolate alcalin ou alcalino-terreux, un hydroxyde alcalin ou alcalino-terreux, ou une amine tertiaire, à une température comprise entre -10 et +80°C et dans un solvant choisi notamment parmi un éther, un éther cyclique, un alcool, un ester, le DMF ou le DMSO.

L'exemple suivant illustre la préparation d'un composé de formule (IIIb)

Exemple CI3' :

Préparation du (5-R,S)-5-méthyl-3-phénylamino-5-((4-phénylthio)phényl)-2-thiooxo-imidazolidin4-one de formule développée suivante:

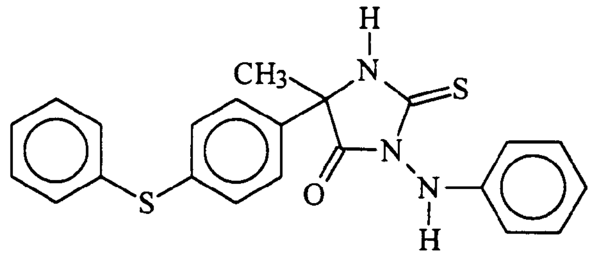

8,48 g (0,0258 mole) de (2-R,S)-2-isothiocyanato-2-((4-phénylthio)phényl)propanoate de méthyle sont dissous dans 250 ml de tétrahydrofuranne. 2,78 g (0,0258 mole) de phénylhydrazine sont coulés goutte à goutte. Le milieu réactionnel est agité pendant 30 minutes à température ambiante, versé dans de l'eau et réextrait à l'éther. Après séchage sur sulfate de magnésium, l'éther est évaporé pour donner 11,46 g d'une huile qui cristallise. Le brut réactionnel est mélangé à l'éther, filtré et recristallisé pour donner 3,36 g (0,0082 mole) de (5-R,S)-5-méthyl-3-phénylamino-5-((4-phénylthio)phényl)-2-thiooxo-imidazolidin-4-one sous forme de cristaux roses fondant à 158 °C avec un rendement de 34%.

Les α-isothiocyanato-esters de formule (VII) sont préparés à partir des esters d'α-aminoacides de formule (VIII), dans laquelle les groupes indiqués ont même signification que dans la formule (VII), selon un des procédés cités dans Sulfur Reports (1989) Volume 8 (5) pages 327-375, soit selon la réaction :

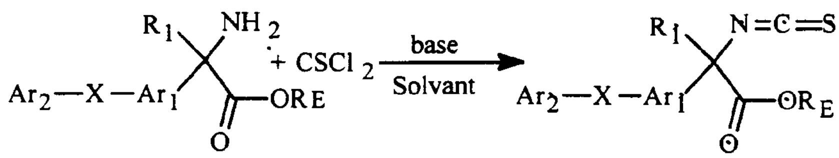


(VIII) (VII)

L'exemple suivant illustre la préparation d'un composé de formule (VII) :

Exemple CI4 :

Préparation du (2-R,S)-2-(4-benzyloxyphényl)-2-isothiocyanatopropanoate de méthyle

A une solution agitée de 84,2 g (1,002 mol) d'hydrogénocarbonate de sodium dans 800ml d'eau, on ajoute 143g (0,501 mol) de (R,S)-(4-benzyloxyphényl)méthyl-glycinate de méthyle, puis 800ml de toluène ; puis on coule goutte à goutte à température ambiante en 1,5 h en contrôlant le dégagement gazeux 40 ml (0,526 mol) de thiophosgène. Aprés 2h d'agitation à température ambiante, décantation de la phase aqueuse, lavage à l'eau, séchage sur sulfate de magnésium et concentration de la phase organique, on isole 155,4g (0,475 mol) de (2-R,S)-2-(4-benzyloxyphényl)-2-isothiocyanatopropanoate de méthyle sous la forme d'une huile trés épaisse avec un rendement de 95%.

En opérant de façon analogue les composés de formule (VII) suivants ont été obtenus :

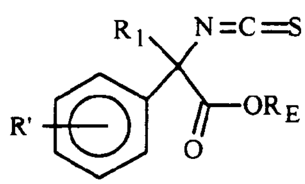

| R' | $R_E$ | $R_1$ | Rdt | pf (°C) |
|---|---|---|---|---|
| 4-BnO | Me | Me | 62% | huile |
| 4-Bn | Me | Me | 50% | huile |
| 4-PhS | Me | Me | 96% | huile |
| 4-PhSO | Me | Me | 100% | huile |
| 4-$PhSO_2$ | Me | Me | 85% | huile |
| 3-BnO | Me | Me | 80% | huile |

Les α-aminoesters de formule (VIII) sont obtenus en estérifiant les α-aminoacides de formule (IX), dans laquelle les groupes indiqués ont même signification que dans la formule (VIII), avec un alcool de formule $R_E$OH, en présence de chlorure de thionyle, procédé décrit dans la littérature par : M.

Brenner et W. Huber : Helv.(1953) Volume 36 Page 1109, selon le schéma :

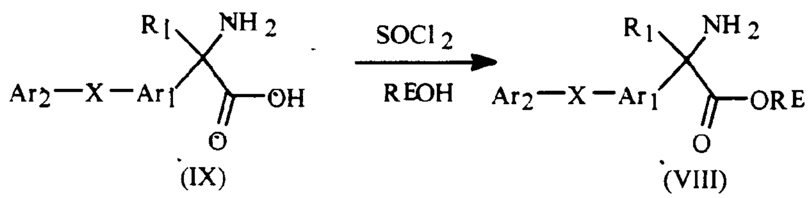


(IX) (VIII)

L'exemple suivant illustre la préparation d'un composé de formule (VIII) :

Exemple CI5 :

Préparation du (R,S)-2-(4-benzyloxyphényl)-2-méthyl-glycinate de méthyle

A une suspension agitée de 38,55 g (0,142 mol) de (R,S)-2-(4-benzyloxyphényl)-2-méthyl-glycine dans 400ml de méthanol maintenue à -20°C, on ajoute progressivement 33,8g (0,284 mol) de chlorure de thionyle. En fin d'addition on laisse remonter à température ambiante la solution obtenue puis on la porte au reflux pendant 16h. Aprés concentration, le résidu est repris à l'eau et la solution obtenue est alcalinisée avec une solution aqueuse concentrée d'hydroxyde de sodium à pH égal à 8. Le précipité est filtré, lavé à l'eau, essoré et séché sous vide. Aprés chromatographie sur 400g de gel de silice en éluant par un mélange heptane/acétate d'éthyle 30/70 et concentration sous pression réduite des fractions intéressantes, on isole 14,7g (0,0515 mol) de (R,S)-2-(4-benzyloxyphényl)-2-méthyl-glycinate de méthyle sous forme de poudre blanche ayant un point de fusion de 67°C avec un rendement de 36%.

Le tableau ci-aprés rassemble des composés de formule (VIII) synthétisés.

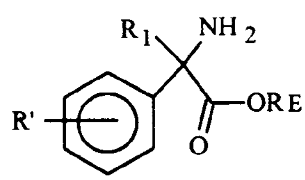

| R' | $R_1$ | $R_E$ | Rdt | pf (°C) ou $n_D$ |
|---|---|---|---|---|
| 4-BnO | Me | Me | 36% | 67°C |
| 4-Bn | Me | Me | 95% | 112-116°C |
| 4-PhS | Me | Me | 66% | 1,6058 |
| 3-BnO | Me | Me | 35% | 50°C |

On décrit à présent une variante de préparation de certains α-aminoesters de formule (VIII), ceux pour

lesquels X représente le groupe $-S(O)_n$ et qui correspondent à la formule (VIIIb).

Les α-aminoesters de formule (VIIIb) sont obtenus par oxydation des α-aminoesters de formule (VIIIa) suivant des procédés décrits dans Advanced Organic chemistry, J. March (1985) Page 1089 et 1090, 3[éme] Edition et selon le schéma :

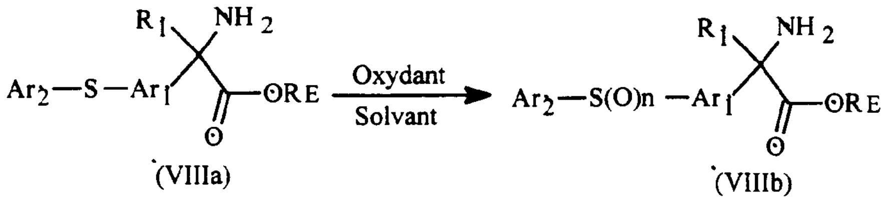


(VIIIa) (VIIIb)

Dans ce schéma n égale 1 ou 2, $R_E$ a la signification indiquée pour la définition de la formule (VII).

Les exemples suivants illustrent la préparation de composés de formule (VIIIb) :

Exemple CI6 :

Préparation du (R,S)-2-amino 2-((4-phénylsulfinyl) phényl) propionate de méthyle:

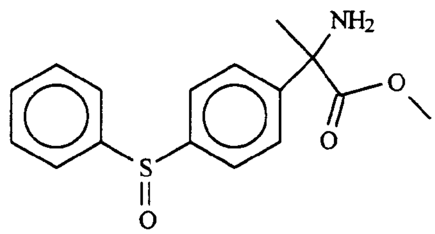

17,22 g (0,06 mole) de 2-amino 2-((4-phénylthio) phényl) propionate de méthyle sont chargés dans 350 ml de dichlorométhane et refroidis à 0°C. 14,1 g (0,06 mole) d'acide métachloroperbenzoïque à 73 % sont ajoutés progressivement en 10 minutes environ. Le milieu réactionnel est agité pendant 2 heures à 0°C. Le précipité est filtré et la phase organique est lavée à la soude normale puis à l'eau et séchée sur sulfate de magnésium. L'évaporation laisse 15 g d'une huile qui est chromatographiée sur silice (éluant : acétate d'éthyle) pour donner 11,3 g (0,037 mol) de (R,S)-2-amino 2-((4-phénylsulfinyl) phényl) propionate de méthyle sous forme d'une huile légèrement jaune ayant un $n_D$ = 1,5979 à 25°C avec un rendement de 62%.

Exemple CI7 :

Préparation du (R,S)-2-amino 2-((4-phénylsulfonyl) phényl) propionate de méthyle:

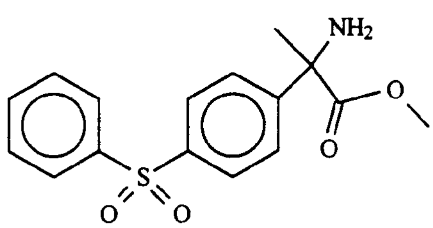

21,52 g (0,075) de (R,S)-2-amino 2-((4-phénylthio) phényl) propionate de méthyle sont chargés dans 400 ml de méthanol et refroidis à 0°C. 50,12 g (0,163 mole) de persulfate de potassium à 49,5 % en solution dans 400 ml d'eau sont ajoutés goutte à goutte en 1 heure à 0°C. Le milieu réactionnel est agité pendant 5 heures à température ambiante. L'oxydant résiduel est détruit par 5 ml d'une solution à 30 % d'hydrogénosulfite de sodium. Le pH du milieu est amené à 8 environ par ajout de 20 g de carbonate de sodium et le solide est filtré. Après agitation du solide dans 500 ml de dichlorométhane, puis filtration du solide résiduel, le filtrat est séché et le solvant évaporé pour donner 16,9 g (0,529 mol) de (R,S)-2-amino 2-((4-phénylsulfonyl) phényl) propionate de méthyle sous forme de cristaux blancs fondant à 124 °C avec un rendement de 70 %.

Les α-aminoacides de formule (IX) sont obtenus par hydrolyse des hydantoïnes correspondantes (X) en milieu acide ou basique selon un procédé analogue à celui décrit dans le brevet néerlandais NE 69 00349 ou dans J. Org. Chem. (1985) Volume 50 Page 1876 à 1878, soit selon le schéma :

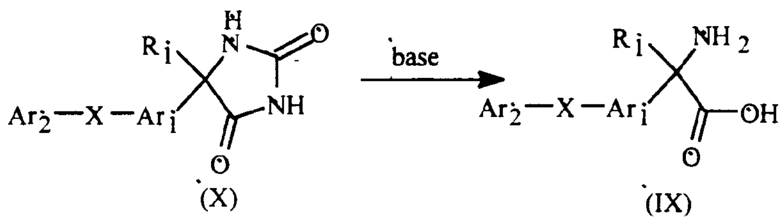


(X) (IX)

L'exemple suivant illustre la préparation de composés de formule (IX).

Exemple CI8 :

Préparation de la (4-R,S)-2-(4-benzyloxyphényl)-2-méthyl-glycine

A une solution agitée de 40g (1 mol) d'hydroxyde de sodium dans 100 ml d'eau, on ajoute 17,2g (0,0506 mol) de (5-R,S)-5-(4-benzyloxyphényl)-5-méthyl-hydantoïne et on porte la suspension obtenue à reflux pendant 24h. Après refroidissement, le milieu réactionel est neutralisé avec une solution aqueuse d'acide chlorhydrique concentré puis la suspension est diluée avec 100 ml d'eau. Le précipité est filtré, lavé à l'eau puis à l'acétone, essoré et séché sous vide. On isole ainsi 16,2 g à 87% molaire par RMN de (R,S)-2-(4-benzyloxyphényl)-2-méthyl-glycine sous forme de poudre blanche ayant un point de fusion supérieur à 300°C.

Le tableau ci aprés rassemble les α-aminoacides de formule (IX) synthétisés:

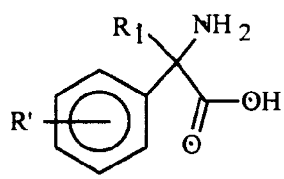

| R' | $R_1$ | Rdt | pf°c ou $n_D$ |
|---|---|---|---|
| 4-(3-Pyridyloxy) | Me | 96% | >300°C |
| 4-BnO | Me | 42% | >300°C |
| 4-Bn | Me | 100% | >300°C |
| 4-PhS | Me | 100% | >300°C |
| 3-BnO | Me | 100% | 270°C |
| 3-PhS | Me | 100% | >300°C |

Les hydantoïnes de formule (X) sont obtenues par réaction de Bücherer-Berg sur les acétophénones correspondantes, procédé décrit dans la littérature : Chem. Rev. (1950) Volume 46 page 422425 ou Chem. Pharm. Bull. (1973) Volume 21 page 685 à 691. Lesdites acétophénones sont aisément accessibles à l'homme du métier.

**Procédé B :**

Selon un mode de réalisation préféré, les composés selon l'invention de formule (II), dans laquelle W représente un oxygène, autrement dit les composés de formule (IIa), sont préparés en faisant réagir successivement le composé de formule (VII) avec une hydrazine de formule $R_3$-NH-$NH_2$, puis avec une base de formule $B^-M^+$, et enfin avec un agent d'alkylation de formule $R_2L$, dans laquelle L et $R_2$ sont définis comme précédemment avec la restriction que $R_2$ est différent de l'atome d'hydrogène.

La base est choisie parmi un alcoolate alcalin ou alcalino-terreux, un hydroxyde alcalin ou alcalino-terreux, ou

une amine tertiaire. La réaction est réalisée à une température comprise entre -10 et +80°C et dans un solvant choisi notamment parmi un éther cyclique ou non cyclique, un alcool, un ester, le DMF, le DMSO, l'acétonitrile ou parmi un aromatique. Le schéma de la réaction est le suivant :

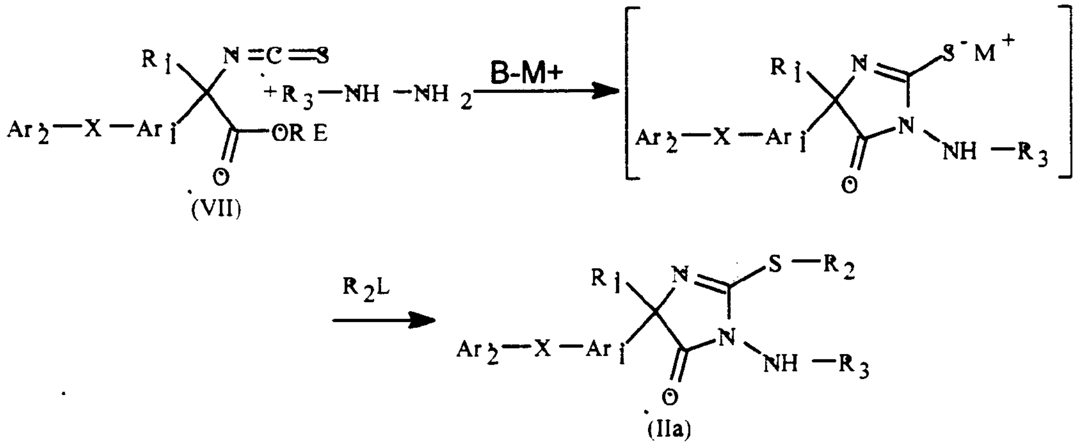


(VII)

(IIa)

L'exemple suivant illustre la préparation de composés de formule (IIa).

Exemple CF2 :

Préparation de la (4-R,S)-4-méthyl-2-méthylthio-4-(4-phénylthiophényl)-1-phénylamino-2-imidazoline-5-one (composé N° 25):

A une solution agitée de 5 g (0,015 mol) de (2-R,S)-2-(4-phénylthiophényl)-2-isothiocyanatopropanoate de méthyle dans 120 ml de tétrahydrofurane anhydre sous couverture d'azote on ajoute 1,62 g (0,015 mol) de phénylhydrazine; on laisse réagir pendant 1h à température ambiante puis on refroidit le milieu réactionnel à 0°C. On ajoute 1,68 g (0,015 mol) de tertiobutylate de potassium, on laisse réagir 0,25 h puis on ajoute 2,13 g (0,015 mol) d'iodure de méthyle. On laisse remonter la température à l'ambiante et on agite pendant 3 h. On verse le milieu réactionnel dans 100 ml d'eau puis on extrait le produit par l'acétate d'éthyle. Après lavage à l' eau, séchage sur sulfate de magnésium et concentration de la phase organique on récupère 7,4 g d'un miel que l'on dissout dans 30 ml d'éther diisopropylique. Le produit précipite à 5°C; après filtration et séchage sous pression réduite, on obtient 4,8 g (76 %) de (4-R,S)-4-méthyl-2-méthylthio-4-(4-phénylthiophényl)-1-phénylamino-2-imidazoline-5-one (composé N° 25) sous forme de cristaux beige fondant à 138°C.

Préparation des composés de formule Ia dans laquelle n = 1 et Y = O :

On décrit à présent la préparation des composés de formule (Ia) pour lesquels n=1 et Y=O et W = O, autrement les composés de formule (XI).

**Procédé C :**

Ces composés sont obtenus en faisant réagir dans un solvant les 2-alkylthio-2-imidazoline-5-ones de formule (II) avec un alcool $R_2OH$ en présence d'une base forte, selon le schéma :

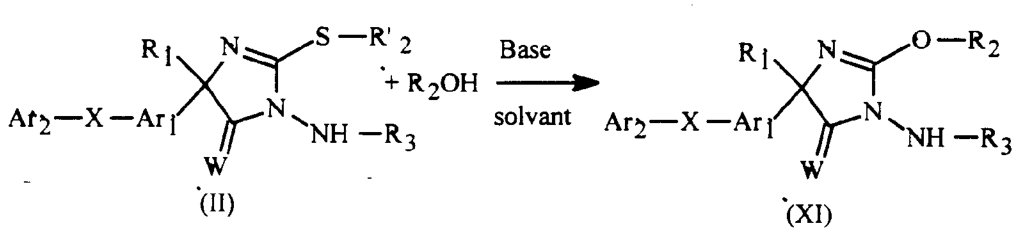


(II)

(XI)

dans lequel $R'_2$ représente un groupe alkyle comprenant de 1 à 3 atomes de carbone.
Comme base forte, on peut utiliser un alcoolate alcalin $R_2O^-M^+$, un hydroxyde alcalin, un hydroxyde alcalino-terreux ou une base organique forte. La réaction est pratiquée de préférence dans l'alcool $R_2OH$, comme sol-

vant, et en utilisant l'alcoolate de sodium correspondant $R_2O^-Na^+$ comme base. La réaction est pratiquée à une température comprise entre 20 et 80°C. Selon une variante du procédé, la réaction est pratiquée en présence d'un oxydant. Comme oxydant on peut utiliser notamment le periodate de sodium ou de potassium.

L'exemple suivant illustre la préparation des composés de formule (XI):

Exemple CF3 :

Préparation de (4-R,S)-4-(4-benzyloxyphényl)-2-méthoxy-4-méthyl-1-phénylamino-2-imidazoline-5-one (composé N° 9):

A une solution de 0,5 g (0,0093 mole) de méthylate de sodium dans 50 ml de méthanol anhydre sous atmosphère inerte, on ajoute 2,6 g (0,0062 mol) de (4-R,S)-4-(4-benzyloxyphényl)-4-méthyl-2-méthylthio-1-phénylamino-2-imidazoline-5-one et 1,3 g de periodate de sodium. On chauffe au reflux pendant 6h. Après refroidissement du milieu réactionnel on neutralise celui-ci avec de l'acide acétique, on le coule dans 100 ml d'eau et on l'extrait par du dichlorométhane. La phase organique est lavée à l'eau, séchée sur sulfate de magnésium puis concentrée sous pression réduite. Le produit brut ainsi obtenu est purifié par chromatographie sur silice (éluant : heptane / acétate d'éthyle 50 / 50). Le produit purifié est cristallisé dans de l'éther diisopropylique. Aprés filtration et séchage sous pression réduite on obtient 0,8g (0,002mol) de (4-R,S)-4-(4-benzyloxyphényl)-2-méthoxy-4-méthyl-1-phénylamino-2-imidazoline-5-one (composé N° 9) sous forme de cristaux blancs ayant un point de fusion de 127°C avec un rendement de 32%.

Préparation des composés de formule Ia dans laquelle n = 0 :

**Procédé D :**

On décrit à présent la préparation des composés de formule (Ia) pour lesquels n=0 et W = O, autrement dit les composés de formule (XII) :

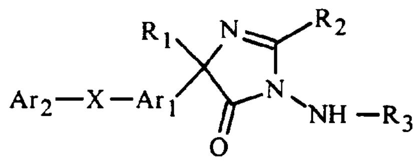

(XII)

Ces composés sont préparés selon le procédé décrit dans la demande internationale WO 94/01410, c'est-à-dire en faisant réagir une hydrazine de formule $R_3$-NH-$NH_2$ sur une azalactone de formule (XIII), selon le schéma réactionnel suivant :

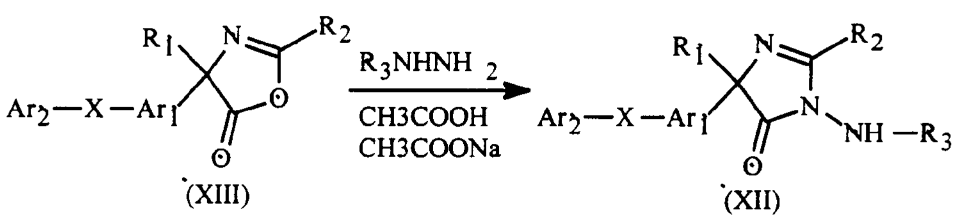


(XIII) (XII)

La réaction est réalisée en chauffant le mélange des réactifs dans l'acide acétique au reflux, éventuellement en présence d'acétate de sodium comme catalyseur.

L'exemple suivant illustre la préparation de composés de formule (XII).

Exemple CI8' :

Préparation du (5-R,S)-2-éthyl-5-méthyl-3-phénylamino-5-((4-phénylthio)phényl)-imidazolidin-4-one: de formule développée :

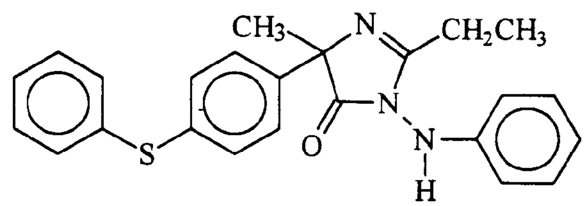

4 g (0,013 mole) de (4-R,S)-2-éthyl-4-méthyl-4-((4-phénylthio)phényl)-3-oxazolin-5-one sont ajoutés à 100 ml d'acide acétique. 1,4 g (0,013 mole) de phénylhydrazine sont coulés goutte à goutte dans le milieu réactionnel. Le milieu réactionnel est chauffé à reflux pendant 2 heures puis l'acide acétique est éliminé sous vide. Le résidu est mélangé à l'eau, neutralisé, puis réextrait au dicholorométhane. Après séchage sur sulfate de magnésium, l'évaporation du solvant laisse 4,09 g d'une huile qui est chromatographiée sur silice (éluant : acétate d'éthyle / heptane 4 / 1) pour donner 3,41 g (0,0085 mole) de (5-R,S)-2-éthyl-5-méthyl-3-phénylamino-5-((4-phénylthio)phényl)-imidazolidin-4-one sous forme d'une huile qui est cristallisée dans l'éther diisopropylique sous forme de cristaux beiges fondant à 90 °C avec un rendement de 66 %.

Les azalactones de formule (XIII) sont obtenues en faisant réagir les α-aminoacides de formule (IX) avec un anhydride d'acide carboxylique, selon un procédé décrit dans la littérature : R. Cotton et Coll., Int. J. Peptide Protein Res. (1986) Volume 28 Page 230 à 244 et selon le schéma :

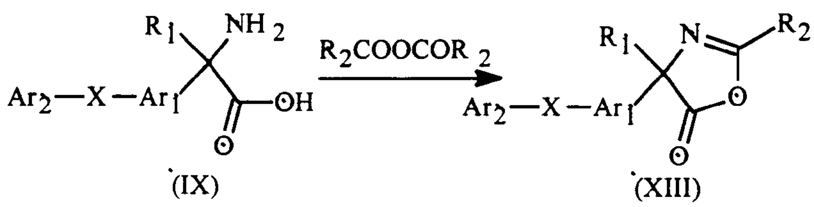


(IX) (XIII)

L'exemple suivant illustre ce procédé pour la préparation des composés de formule (XIII).

Exemple CI9 :

Préparation du (4-R,S)-2-éthyl-4-méthyl-4-((4-phénylthio)phényl)-3-oxazolin-5-one de formule développée :

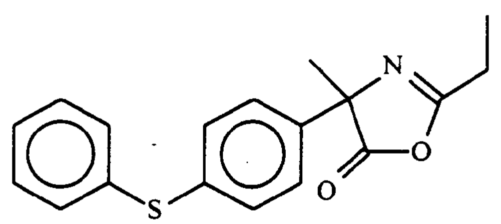

14,2 g (0,052 mole) d'acide (2-R,S)-2-amino-2-((4-phénylthio)phényl) propionique sont mis en suspension dans 200 ml d'anhydride propionique. Le milieu est chauffé à reflux pendant 1 heure puis l'anhydride propionique est chassé sous vide de pompe par azéotropie par ajout d'heptane. Le résidu (16 g) est dissous à chaud dans 100 ml d'éthanol. Un solide blanc cristallise (3,0 g) qui est filtré et écarté. Les jus sont reconcentrés pour donner 12,3 g d'une huile brune qui est chromatographiée sur silice (éluant : acétate d'éthyle / heptane 4 / 1) pour donner 4,76 g (0,0153 mole) de (4-R,S)-2-éthyl-4-méthyl-4-((4-phénylthio)phényl)-3-oxazolin-5-one sous forme d'huile (RMN 250 MHz - $CHCl_3$ - en ppm : 1,3 (t,3H); 1,75 (s,3H); 2,58 (q,2H); 7,2 à 7,6 (m,9H)) avec un rendement de 29 %.

On décrit à présent un second mode de réalisation du composé de formule Ia selon l'invention, selon lequel on introduit un groupe $Ar_2$-X sur une imidazolinone de formule (XIV) :

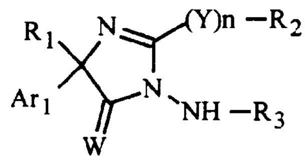

(XIV)

Ce second mode de réalisation correspond aux procédés E, F, G, H, I, J, K, L, M qui sont définis ci-après.

**Procédé E :**

On décrit tout d'abord la préparation des composés de formule générale (Ia) :

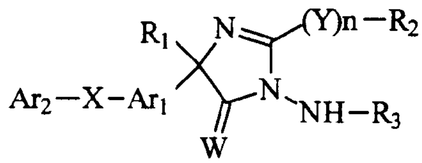

(Ia)

dans laquelle X a la même signification qu'un groupe X', qui est défini ci-dessous dans la description du procédé E.

Les composés de formule générale (Ia) sont obtenus par condensation d'un dérivé organométallique d'une imidazolinone de formule (XV) avec un radical électrophile $Ar_2$-$X'^+$ selon le schéma réactionnel :

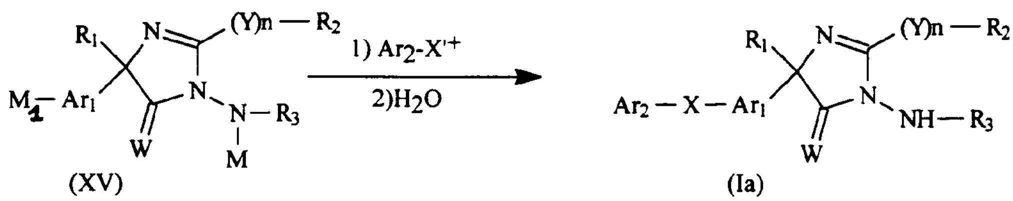


(XV) (Ia)

dans lequel $M_1$ représente un métal choisi parmi le lithium, le sodium, le magnésium, le zinc, le titane, l'étain, le cuivre, le bore ou le silicium et est de préférence le lithium, et dans lequel X' est tel que $Ar_2$-X' est un radical électrophile généré par un composé choisi notamment parmi :

- un halogénure ou un sulfate d'aralkyle de formule $Ar_2$-$R_{12}$-L
  dans laquelle :
  - $R_{12}$ représente une chaine aliphatique de 1 à 6 atomes de carbone, linéaire ou ramifiée, saturée ou insaturée éventuellement substituée par un halogène ou un groupe alkoxy ou un radical oxo ; et
  - L a la même signification que précédemment ;
- un composé carbonylé de formule :

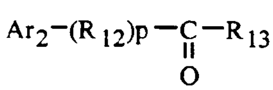

  dans laquelle :
  - p = 0 ou 1 ;
  - $R_{12}$ a la même signification que ci-dessus ; et
  - $R_{13}$ représente l'atome d'hydrogène, un groupe $C_1$-$C_6$ alkyle, un halogène, un groupe alkoxy, aryloxy, ou dialkylamino ;
- un chloroformiate de formule :

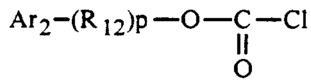

dans laquelle p et $R_{12}$ ont les mêmes significations que ci-dessus ;

- un isocyanate ou un isothiocyanate de formule :

$$Ar_2\text{-}(R_{12})p\text{-}N{=}C{=}Q$$

dans laquelle p et $R_{12}$ ont les mêmes significations que ci-dessus, et Q représente l'atome d'oxygène ou de soufre ;

- un halogénure de sulfonyle de formule :

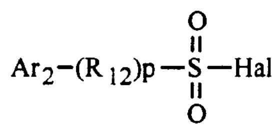

dans laquelle $R_{12}$ et p ont les mêmes significations que précédemment et Hal représente un atome d'halogène, de préférence l'atome de chlore ;

- un époxyde de formule :

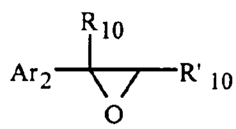

Lorsque, selon une variante préférée de l'invention, la lettre $M_1$ de la formule (XV) représente le lithium, les composés de formule (XV) correspondants, soit les organolithiens de formule (XVII), sont obtenus à partir des dérivés halogénés (XVI), dans lesquels Hal représente un atome d'halogène, de préférence le chlore ou le brome, par échange métal halogène (Hal) comme décrit par R.G. JONES et H. GILMAN, Organic Réactions (19), volume 6, page 339. La réaction est la suivante :

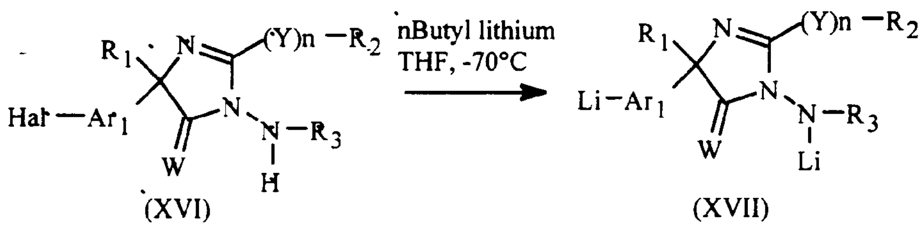


(XVI) (XVII)

Lorsque la lettre $M_1$ de la formule (XV) ne représente pas le lithium, les organométalliques de formule (XV) correspondants peuvent être obtenus à partir des organolithiens (XVII) par réaction de transmétallation comme décrit dans Advanced organic chemistry , J. March, (1985), p. 557, 3ème Edition.

L'exemple suivant illustre la préparation d'un composé de formule (Ia).

Exemple CF4 :

Préparation de (4-R,S)-4-[4-((4-chlorophényl)hydroxyméthyl)phényl]-4-méthyl-2-méthylthio-1-phénylamino 2-imidazoline-5-one (composé N° 30):

1 g (0,0025 mol) de (4-R,S)-4-(4-bromophényl)-4-méthyl-2-méthylthio-1-phénylamino 2-imidazolin-5-one est dissous sous atmosphère inerte dans 20 ml de THF anhydre puis le milieu réactionnel est refroidi à -70°C. A cette température sont ajoutés 2 équivalents de n-butyllithium (3,2ml de solution hexanique 1,6 M). Après 15 mn d'agitation à -70°C 0,36g (0,0025 mol) de 4-chlorobenzaldéhyde est ajouté en solution dans 3 ml de THF. Après 30 mn de réaction le milieu réactionnel est hydrolysé à -70°C avec une solution saturée de chlorure d'ammonium. Après extraction à l'acétate d'éthyle, lavage des extraits organiques avec une solution saturée

de chlorure de sodium, séchage, évaporation des solvants, et chromatographie sur colonne de silice du produit brut (Acétate d'éthyle / Heptane : 50 / 50), on récupère 0,95g (0,002 mol) d'un solide blanc qui est la (4-R,S)-4-[4-((4-chlorophényl)hydroxyméthyl)phényl]-4-méthyl-2-méthylthio-1-phénylamino 2-imidazoline-5-one, ayant un point de fusion de 94°C avec un rendement de 82%.

Les 2-imidazoline-5-ones de formule (XVI) sont obtenues par un des procédés décrits dans la demande de brevet européen EP 0 551048 publiée le 14 Juillet 1993, dans le cas où Y=S et n=1 ; selon un procédé analogue au procédé C décrit précédemment dans le cas où n égale 1 et Y signifie O ; soit selon un procédé analogue au procédé D décrit précédemment lorsque n égale 0.

**Procédé F :**

On décrit à présent la préparation des composés de formule générale (Ib) :

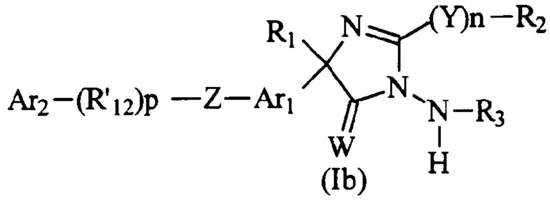

(Ib)

dans laquelle p et Z ont les mêmes significations que précédemment, et $R'_{12}$ représente une chaine aliphatique de 1 à 6 atomes de carbone, linéaire ou ramifiée, saturée ou insaturée.

Ces composés sont obtenus selon des procédés connus en faisant réagir une imidazolinone de formule (XVI) avec un nucléophile de formule (XVIII) selon un procécé analogue à celui cité dans Comprehensive heterocyclic chemistry, A. R. Katritzky, Volume 2, Part 2A, Page 359, soit selon le schéma :

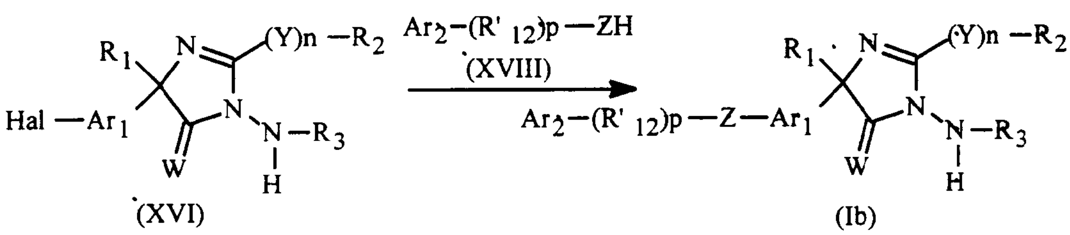


(XVI) (Ib)

La réaction est pratiquée dans les conditions usuellement mises en oeuvre dans les réactions de substitution aromatique.

L'exemple suivant illustre la préparation de composés de formule (Ib).

<u>Exemple CF5</u> :

Préparation de (4-R,S)-4-[6-(4-fluorophénylthio)-5-nitropyridine-2-yl]-4-méthyl-2-méthylthio-1-phénylamino-2-phénylamino-2-imidazoline-5-one (Composé N° 64 ):

A 1,00 g (2,5 mmol) de 4-(6-chloro-5-nitro-pyridin-2-yl)-4-méthyl-2-méthylthio-1-phénylamino-2-imidazoline-5-one en solution dans 50 ml d'acétone sous argon sont ajoutés à température ambiante 1g de $K_2CO_3$ puis 0,27 ml (2,5 mmol) de 4-fluorothiophénol. Après 2 heures de réaction à température ambiante, le milieu est dilué à l'eau; le produit est extrait à l'acétate d'éthyle; les extraits sont séchés sur sulfate de magnésium anhydre puis concentrés sous pression réduite. Le produit après purification chromatographique sur silice (Eluant : acétate d'éthyle / Heptane 20 / 80) est cristallisé dans l'éther diisopropylique. On récupère après filtration et séchage 0,8g (1,66 mmol) de (4-R,S)-4-[6-(4-fluorophénylthio)-5-nitropyridine-2-yl]-4-méthyl-2-méthylthio-1-phénylamino-2-imidazoline-5-one (Composé N° 64 ) sous forme de cristaux ayant un point de fusion de 171°C avec un rendement de 66%.

**Procédé G :**

On décrit à présent la préparation des composés de formule (Ic) :

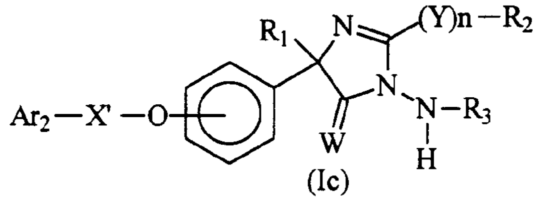

(Ic)

dans laquelle X' a la même signification que précédemment.

Les composés de formule (Ic) sont obtenus par réaction d'une 4-(hydroxyphényl)-2-imidazoline-5-one, de formule (XIX), avec un radical électrophile $Ar_2$-$X'^{+}$ selon le schéma réactionnel :

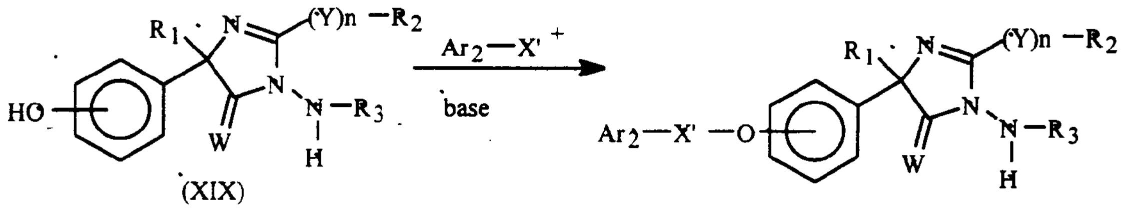


(XIX)

La réaction est effectuée en milieu basique dans les conditions usuellement pratiquées pour la condensation entre les phénols et les différents radicaux électrophiles (procédés connus en soi dans la littérature). Comme base on peut utiliser, notamment, les carbonates et les hydroxydes alcalins ou alcalinoterreux, les bases organiques. La réaction est pratiquée à une température comprise entre +20°C et + 150°C. Elle est conduite dans des solvants tels que les alcools, les cétones, l'acétonitrile, les esters aliphatiques, les éthers, les éthers cycliques, les solvants aromatiques, les solvants chlorés, le diméthylformamide, le diméthylsulfoxyde, la N-méthylpyrolidone.

L'exemple suivant illustre la préparation de composés de formule (Ic).

Exemple CF6 :

Préparation de la (4-R,S)-4-méthyl-4-(4-(3-méthylbenzyloxy)phényl)-2-méthylthio-1-phénylamino-2-imidazoline-5-one (composé N° 22):

A une solution agitée de 2g (0,0061 mol) de (4-R,S)-4-(4-hydroxyphényl)-4-méthyl-2-méthylthio-1-phénylamino-2-imidazoline-5-one dans 50 ml d'éthanol, on ajoute 0,92g (0,0067 mol) de carbonate de potassium 0,94 g (0,0067 mol) de chlorure de 3-méthylbenzyle et 0,1g (0,00061 mol) d'iodure de potassium. On porte le milieu réactionnel au reflux pendant 8h. On verse celui-ci dans 150 ml d'eau. Le précipité formé est lavé avec une solution aqueuse 1N d'hydroxyde de sodium, puis à l'eau jusqu'à neutralité, essoré, lavé à l'éther diisopropylique puis séché sous pression réduite.On obtient ainsi 1,94g (0,0045 mol) de (4-R,S)-4-méthyl-4-(4-(3-méthylbenzyloxy)phényl)-2-méthylthio-1-phénylamino-2-imidazoline-5-one (composé N° 22) sous forme de cristaux blanc ayant un point de fusion de 151°C avec un rendement de 75%.

Les composés hydroxylés de formules (XIX) sont obtenus par à partir des composés benzylés (Id) correspondants, en retirant le groupe benzyle au moyen d'une réaction dite de débenzylation. La débenzylation est effectuée suivant des procédés usuellement utilisés, soit par un hydracide, soit par hydrogénation catalytique,et connus dans la littérature : G. Büchi et S. M. Weinreb, J. Am. Chem. Soc. (1971) Volume 93 Page 746 ou O. Th. Schmidt et H. Schmadel, Annalen (1961) Volume 649 Page 149. Le schéma réactionnel est le suivant :

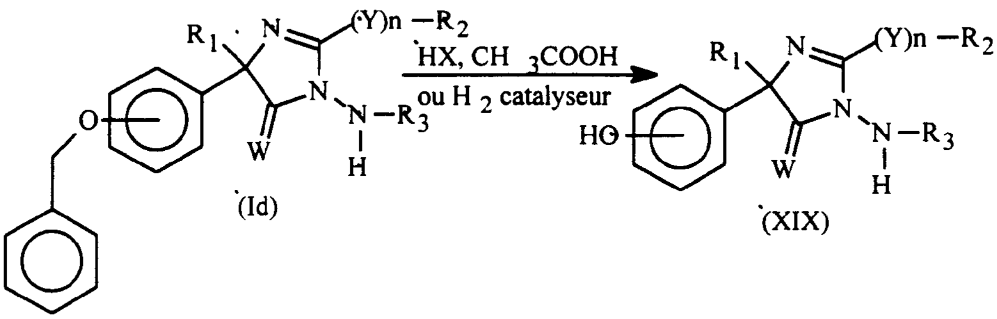


(Id) (XIX)

L'exemple suivant illustre ce procédé pour la préparation des composé de formule (XIX).

Exemple CI10 :

Préparation de la (4-R,S)-4-(4-hydroxyphényl)-4-méthyl-2-méthylthio-1-phénylamino-2-imidazoline-5-one, de formule développée :

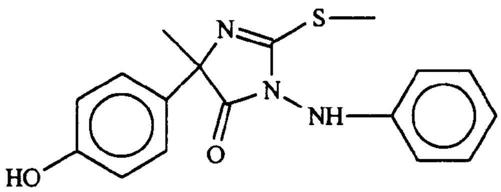

A une suspension maintenue agitée de 31,2 g (0,075 mol) de (4-R,S)-4-(4-benzyloxyphényl)-4-méthyl-2-méthylthio-1-phénylamino-2-imidazoline-5-one dans 370 ml d'acide acétique on ajoute 92 ml d'acide chlorhydrique concentré (d =1,18) et l'on chauffe à 75°C pendant 2h. Après avoir refroidi le milieu réactionel à température ambiante, on verse celui-ci dans 500 ml d'eau glacée puis on neutralise le milieu avec une solution aqueuse concentrée d'hydroxyde de sodium. Le précipité formé est agité 1h à 5°C puis filtré, lavé à l'eau, essoré et séché sous pression réduite. On obtient ainsi 22g (0,067 mol) de (4-R,S)-4-(4-hydroxyphényl)-4-méthyl-2-méthylthio-1-phénylamino-2-imidazoline-5-one sous forme de cristaux blancs ayant un point de fusion de 206°C avec un rendement de 90%.

Le tableau ci aprés rassemble les imidazolinones de formule (XIX) synthétisées:

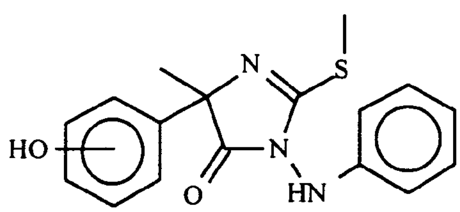

| Position OH | Rdt (%) | pF (°C) |
|---|---|---|
| 3 | 68 | 159 |
| 4 | 90 | 206 |

Les 4-(Benzyloxyphényl)-2-imidazoline-5-ones (Id) sont obtenues selon un des procédés décrits précédemment.

**Procédé H :**

On décrit à présent la préparation des composés de formule (Ie) :

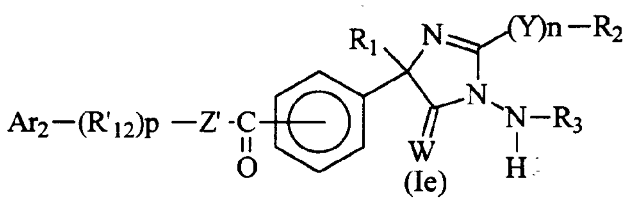

(Ie)

dans laquelle p et R'12 ont la même signification que précédemment et Z' représente l'atome d'oxygène,de soufre, ou le groupe N-$R^{14}$, dans lequel $R_{14}$ représente l'atome d'hydrogène ou un groupe $C_1$-$C_6$ alkyle.

Ces composés sont obtenus en faisant réagir des dérivés de 4-(carboxyphényl)-2-imidazoline-5-one de formule (XX), avec un agent de chloration tel que le chlorure de thionyle, le chlorure d'oxalyle ou le phosgène, éventuellement en présence d'un catalyseur tel que le diméthylformamide et dans un solvant inert ; puis avec un composé nucléophile de formule : $Ar_2$-($R'^{12}$)p-Z'H (XVIII), en présence d'une base telle qu'un carbonate alcalin ou alcalinoterreux ou d'une base organique, selon le schéma :

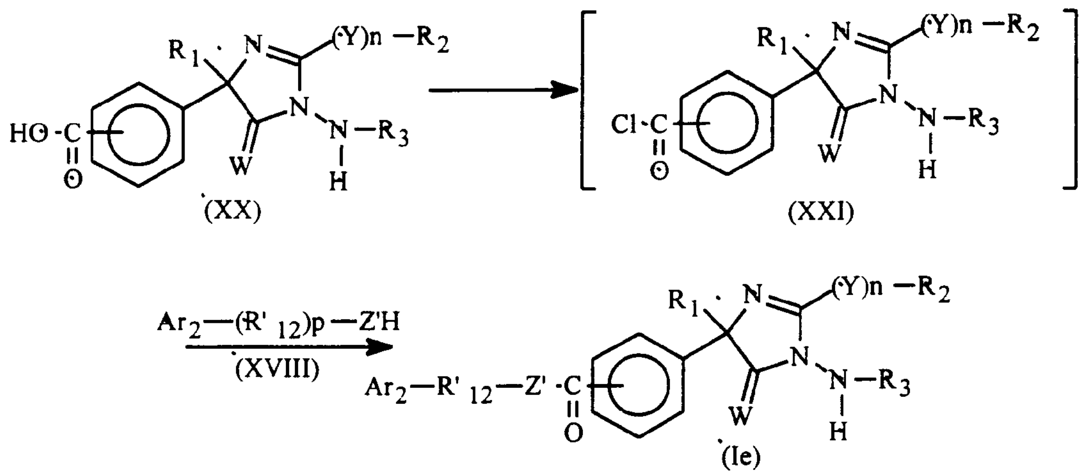


(XX) (XXI) (Ie)

L'exemple suivant illustre ce procédé pour la préparation des composés de formule (Ie).

Exemple CF7 :

Préparation de la (4-R,S)-4-(4-(4-chlorophénylaminocarbonyl)phényl)-4-méthyl-2-méthylthio-1-phénylamino-2-imidazoline-5-one (composé N° 45):

1g (0,0028 mol) de (4-R,S)-4-(4-carboxyphényl)-4-méthyl-2-méthylthio-1-phénylamino-2-imidazoline-5-one est dissous dans 20 ml de dichloro-1,2-éthane à température ambiante. A cette température sont ajoutés 0,5 ml (0,0056 mol) de chlorure d'oxalyle puis une goutte de diméthylformamide. A la fin du dégagement gazeux, le solvant est évaporé et le chlorure d'acide est repris dans 20 ml de THF anhydre. A température ambiante sont ensuite ajoutés 0,4 ml (0,0028 mol) de triéthylamine puis 0,71g (0,0056 mol) de 4-chloroaniline. Après 1h d'agitation le milieu est hydrolysé. Après extraction à l'acétate d'éthyle, lavage des phases organiques avec une solution saturée de chlorure de sodium, séchage, évaporation des solvants 1g d'huile brune est récupéré. Après cristallisation dans l'éther diisopropylique, filtration et séchage on isole 0,85 g (0,0018 mol) de (4-R,S)-4-(4-(4-chlorophénylaminocarbonyl)phényl)-4-méthyl-2-méthylthio-1-phénylamino-2-imidazoline-5-one sous forme d'un solide beige ayant un point de fusion de 210°C avec un rendement de 65%.

Les 4-(carboxyphényl)-2-imidazoline-5-ones de formule (XX) sont préparés en faisant réagir un organolithien de formule (XVII) avec du gaz carbonique selon un procédé analogue à celui cité dans Advanced organic chemistry, J. March, (1985) Page 826 3ème Edition, autrement dit selon la réaction :

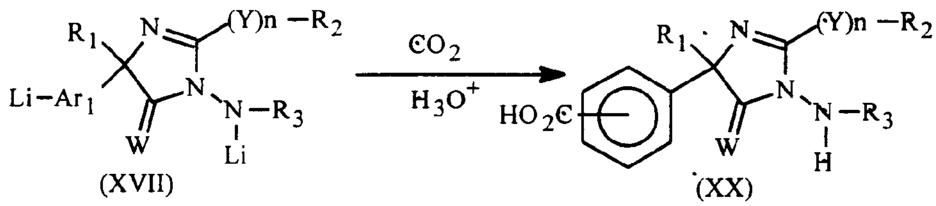


(XVII) (XX)

La réaction est effectuée en faisant barbotter du gaz carbonique dans le milieu réactionnel contenant l'organolithien.

L'exemple suivant illustre ce procédé pour la préparation des composés de formule (XX).

Exemple CI11 :

Préparation de (4-R,S)-4-(4-carboxyphényl)-4-méthyl-2-méthylthio-1-phénylamino 2-imidazolin-5-one, de formule développée :

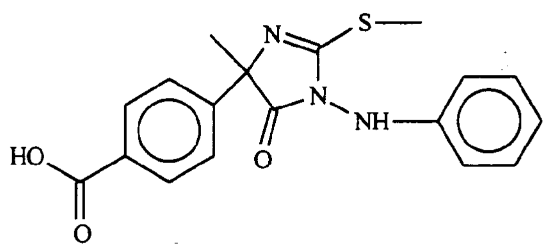

1g (0,0025 mol) de (4-R,S)-4-(4-bromophényl)-4-méthyl-2-méthylthio-1-phénylamino 2-imidazolin-5-one est dissous sous atmosphère inerte dans 20 ml de tétrahydrofurane anhydre. Le milieu réactionnel est refroidi à -70°C. A cette température sont ajoutés 2 équivalents de n-butyllithium (3,2 ml de solution hexanique1,6 M). Après 15 mn d'agitation à -70°C, on fait barboter le dioxyde de carbone sec. Après 30 mn, le milieu réactionnel est hydrolysé à -70° C. Après extraction à l'acétate d'éthyle puis lavage de la phase organique avec une solution saturée de carbonate de potassium, les phases aqueuses sont réunies puis acidifiées. Après extraction à l'acétate d'éthyle, séchage, évaporation des solvants, on récupère 0,5 g (0,0014 mol) de (4-R,S)-4-(4-carboxyphényl)-4-méthyl-2-méthylthio-1-phénylamino 2-imidazolin-5-one sous forme d'une poudre blanche fondant à 183°C avec un rendement de 56%.

**Procédé I :**

On décrit à présent la préparation des composés de formule générale (If) :

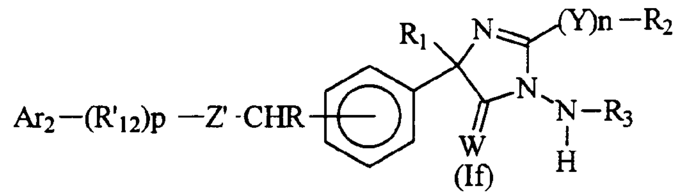

(If)

dans laquelle R' 12, p et Z' ont la même signification que précédemment, et R represente un atome d' hydrogène, un radical alkyl, haloalkyl, alkoxyalkyl ou alkylidène , chacun de ces radicaux alkyle comprenant de 1 à 6 atomes de carbone. R conserve cette même définition dans le reste de la description.

Ces composés sont obtenus par réaction d'un composé nucléophile de formule $Ar_2$-$(R'_{12})_p$-Z'H (XVIII) avec une 4-(chlorométhylphényl)-2-imidazoline-5-one de structure (XXIII).

La réaction est effectuée en présence d'une base dans les conditions usuellement pratiquées pour la condensation entre les alcools et différents électrophiles et connus dans la littérature : Advanced organic chemistry, J. March, (1985) Page 342 3ème Edition, autrement dit selon le schéma réactionnel :

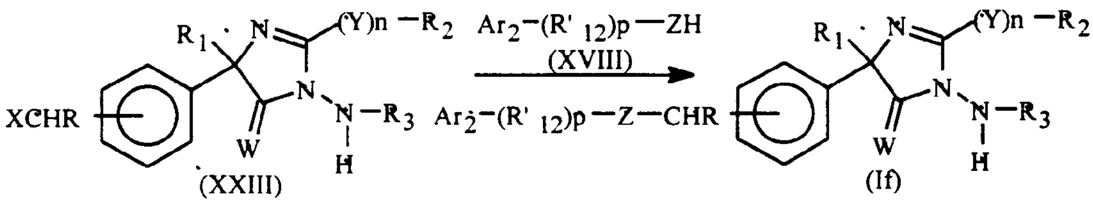

(XXIII) (If)

Comme base, on peut utiliser les carbonates et les hydroxydes alcalins ou alcalinoterreux ou les bases organiques. La réaction est pratiquée à une température comprise entre 20°C et 150°C. La réaction est conduite dans les solvants tels que les alcools, l'acétonitrile, les esters aliphatiques, les éthers, les éthers cycliques, les solvants aromatiques, les solvants chlorés, les cétones, le diméthylformamide, le diméthylsulfoxyde, la N-méthylpyrolidone.

L'exemple suivant illustre ce procédé pour la préparation des composés de formule (If).

Exemple CF8 :

Préparation de (4-R,S)-4-(4-((2,4-difluorophénoxy)méthyl)phényl)-4-méthyl-2-méthylthio-1-phénylamino 2-imidazoline-5-one (composé N° 47)

0,3 g (0,0083 mol) de (4-R,S)-4-(4-chlorométhyl)phényl)-4-méthyl-2-méthylthio-1-phénylamino 2-imidazoline-5-one est mélangé dans le DMF avec 0,11 g (0,0083 mol) de 2,4-difluorophénol, 0,12 g (0,0083 mol) de carbonate de potassium et 0,14 g (0,0083 mol) de iodure de potassium. Le mélange réactionnel est porté à 80°C pendant 2h. Après hydrolyse à l'eau, extraction à l'acétate d'éthyle, lavage des extraits organiques avec une solution saturée de chlorure de lithium, séchage et évaporation des solvants, le miel obtenu est cristalisé dans l'éther diisopropylique puis filtré. On récupère ainsi 0,250 g (0,0055 mol) de (4-R,S)-4-(4-((2,4-difluorophénoxy)méthyl)phényl)-4-méthyl-2-méthylthio-1-phénylamino 2-imidazoline-5-one sous la forme d'un précipité beige fondant à 152°C avec un rendement de 66%.

Les 4-(chlorométhylphényl)-2-imidazoline-5-one de structure (XXIII) sont obtenues par halogénation des 4-(hydroxyméthylphényl)-2-imidazoline-5-one de structure (XXII). L'halogénation est effectuée suivant des procédés usuellement utilisés pour ce type de réaction à l'aide d'hydracide ou d'halogénure d'acide inorganique tel que $SOCl_2$, $PCl_5$, $PCl_3$, $POCl_3$ comme cité dans Advanced organic chemistry, J. March, (1985), Page 382, 3 ème Edition et selon le schéma réactionnel :

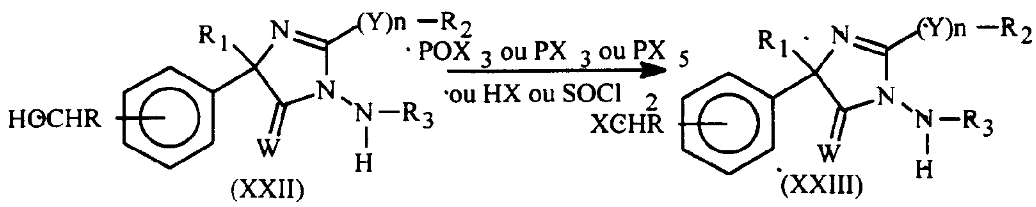

(XXII) (XXIII)

L'exemple suivant illustre ce procédé pour la préparation des composés de structure (XXIII):

Exemple CI12 :

Préparation de (4-R,S)-4-(4-chlorométhylphényl)-4-méthyl-2-méthylthio-1-phénylamino 2-imidazoline-5-one, de formule développée :

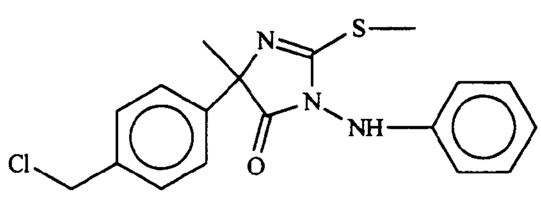

A une solution de 0,9 g (0,0026 mole) de (4-R,S)-4-(4-hydroxyméthylphényl)-4-méthyl-2-méthylthio-1-phénylamino 2-imidazoline-5-one dans 10 ml de toluène, sont ajoutés à température ambiante 0, 22 ml (0,0026

mole) de pyridine puis 0, 2 ml (0,0026 mole) de chlorure de thionyle en solution dans 1 ml de toluène. Le mélange réactionnel est porté à 60°C pendant 3h puis refroidi et hydrolysé. Après extraction à l'acétate d'éthyle, lavage des extraits organiques avec une solution saturée de chlorure de sodium, séchage et évaporation des solvants, un miel jaune est obtenu. Après chromatographie sur colonne de silice (Acétate d'éthyle/ Heptane : 50/ 50), on récupère 0,36g (0,001 mole) de (4-R,S)-4-(4-chlorométhylphényl)-4-méthyl-2-méthylthio-1-phénylamino 2-imidazoline-5-one sous la forme d'une poudre jaune fondant à 130°C avec un rendement de 38%.

Les (hydroxyméthyl)phényl-2-imidazoline-5-ones de formules (XXII) sont obtenues par réduction des composés carbonylés (XXIV) correspondants. Cette réduction peut être effectuée suivant des procédés usuellement utilisés pour ce type de réaction, décrit dans la littérature: Advanced organic chemistry, J. March, (1985) Pages 1093-6 3ème Edition et selon le schéma :

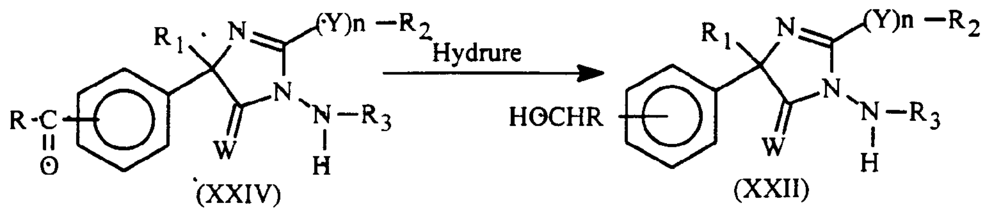


(XXIV) (XXII)

L'exemple suivant illustre ce procédé pour la préparation des composés de structure (XXII) :

Exemple CI13 :

Préparation de (4-R,S)-4-(4-hydroxyméthylphényl)-4-méthyl-2-méthylthio-1-phénylamino 2-imidazoline-5-one, de formule développée :

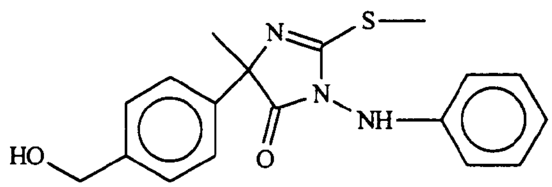

A une solution de 9 g (0,0026 mole) de (4-R,S)-4-(4-formylphényl)-4-méthyl-2-méthylthio-1-phénylamino 2-imidazoline-5-one dans 100 ml d'éthanol absolu à 0°C sont ajoutés 1,02 g (0,0026 mole) de $NaBH_4$. Après 4h d'agitation à température ambiante, le milieu est hydrolysé puis extrait à l'acétate d'éthyle. Les extraits organiques sont lavés avec une solution saturée de chlorure de sodium, séchés, évaporés et chromatographiés sur colonne de silice (Acétate d'éthyle/ Heptane : 50/ 50).on récupère 0,9 g (0,00026 mole) de (4-R,S)-4-(4-hydroxyméthylphényl)-4-méthyl-2-méthylthio-1-phénylamino 2-imidazoline-5-one sous la forme d'une poudre marron fondant à 146°C, avec un rendement de 10%.

Le tableau ci aprés rassemble les imidazolinones de formule (XXII) synthétisées:

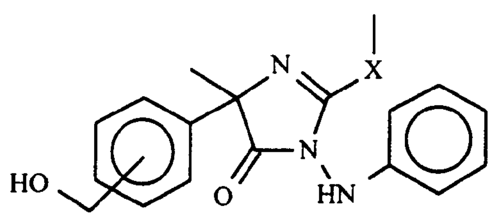

| Position OH | X | Rdt (%) | pF (°C) |
|---|---|---|---|
| 3 | S | 76 | 147 |
| 4 | S | 10 | 146 |
| 3 | O | 76 | 157 |

Les composés carbonylés de formule (XXIV) sont préparés par réaction d'un organolithien de formule (XVII) avec un composé électrophile carbonylé comme décrit par J. EVANS, J. Chem. Soc, (1956), page 4691 et selon le schema réactionnel :

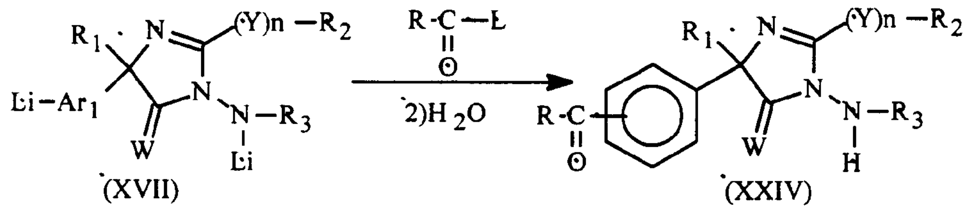


(XVII) (XXIV)

L'exemple suivant illustre ce procédé pour la préparation des composés de structure (XXIV ):

Exemple CI14 :

Préparation de (4-R, S)-4-(4-formylphényl)-4-méthyl-2-méthylthio-1-phénylamino-2-imidazoline-5-one, de formule développée :

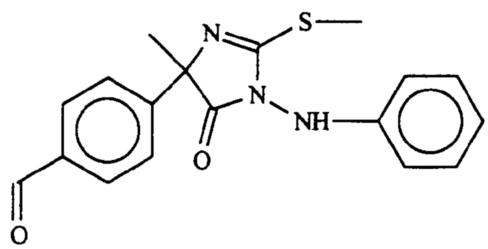

1 g (0,0025 mole) de 4-(4-bromophényl)-4-méthyl-2-méthylthio-1-phénylamino-2-imidazoline-5-one est dissous sous atmosphère inerte dans 20 ml de THF anhydre puis porté à -70°C. A cette température sont ajoutés 2 equivalents de nBuli (3,2 ml; 1,6M). Après 15 mn d'agitation à -70°C, 0,2 ml (0,0025 mole) de diméthylformamide est ajouté en solution dans 1 ml de THF. Après 30 mn, le milieu est hydrolysé à -70°C avec une solution saturée de chlorure d'ammonium. Après extraction à l'acétate d'éthyle, lavage des phases organiques avec une solution saturée de chlorure de sodium, séchage, évaporation des solvants, et chromatographie sur colonne de silice du produit brut (Acétate d'éthyle / Heptane : 50 / 50) 0,55g d'huile jaune claire précipitant dans l'éther isopropylique est obtenue. On récupère ainsi 0,35 g (0,001) de (4-R, S)-4-(4-formylphényl)-4-méthyl-2-méthylthio-1-phénylamino-2-imidazoline-5-one sous forme d'une poudre blanche fondant à 181°C, soit un rendement 40%.

Le tableau ci aprés rassemble les imidazolinones de formule (XXIV) synthétisées:

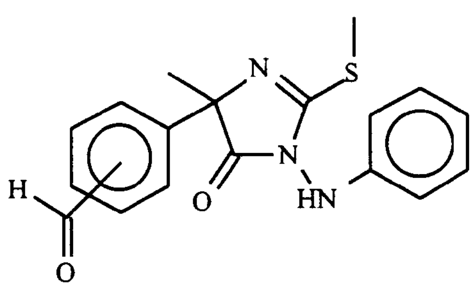

| Position HCO- | Rdt (%) | pF (°C) |
|---|---|---|
| 3 | 48 | 157 |
| 4 | 40 | 181 |

**Procédé J :**

On décrit à présent la préparation des composés de formule (Ig) :

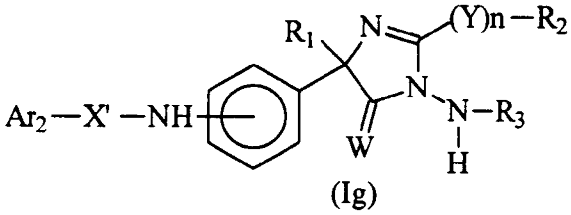

(Ig)

dans laquelle X' a la même signification que précédemment.

Ces composés sont obtenus par réaction d'une 4-(aminophényl)-2-imidazoline-5-one de formule (XXV) avec un radical électrophile de formule $Ar_2X'^+$, tel que défini précédemment, selon le schéma réactionnel :

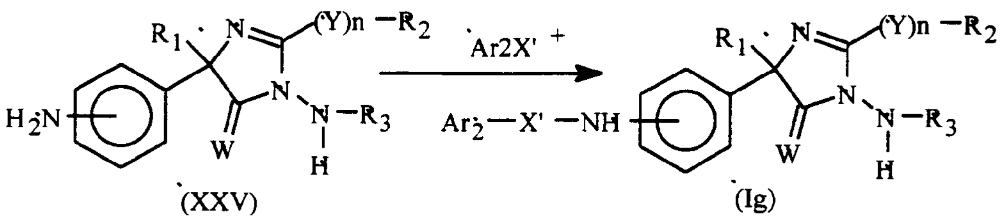


(XXV) (Ig)

La réaction est effectuée dans les conditions usuellement pratiquées pour la condensation entre les anilines et les différents composés électrophiles. La réaction est effectuée en présence d'une base. Comme base on peut utiliser les carbonates, les hydroxydes alcalins ou alcalinoterreux, les bases organiques. La réaction est pratiquée à une température comprise entre 20°C et 150°C, et est conduite dans un solvant tel que : les alcools, l'acétonitrile, les esters aliphatiques, les éthers, les éthers cycliques, les solvants aromatiques, les solvants chlorés, le diméthylformamide, le diméthylsulfoxyde, la N-méthylpyrolidone.

L'exemple suivant illustre ce procédé pour la préparation des composés de formule (Ig).

Exemple CF9 :

Préparation de (4-R,S)-4-(4-benzoylaminophényl)-4-méthyl-2-méthylthio-1-phénylamino-2-imidazoline-5-one (composé N° 1):

A une solution de 2 g (6,1 mmoles) de (4-R,S)-4-(4-aminophényl)-4-méthyl-2-méthylthio-1-phénylamino-2-imidazoline-5-one et de 0,62 g (6,1 mmoles) de triéthylamine dans 50 ml de tétrahydrofurane on ajoute 0,86 g (6,1 mmoles) de chlorure de benzoyle. On laisse réagir 2h à température ambiante, on filtre le précipité de chlorhydrate de triéthylamine puis on concentre le filtrat sous pression réduite. On fait cristalliser le produit brut dans l'éther diéthylique. On récupère ainsi 2,47 g (5,7 mmoles) de (4-R,S)-4-(4-benzoylaminophényl)-4-méthyl-2-méthylthio-1-phénylamino-2-imidazoline-5-one (composé N° 1) à l'état de solide blanc fondant à 212°C avec un rendement de 94%.

Les 4-(aminophényl)-2-imidazoline-5-ones de formule (XXV) sont préparées par réduction des 4-(nitrophényl)-2-imidazoline-5-ones de formule (XXVI). La réduction est effectuée selon les méthodes usuelles de réduction des groupes nitro comme, par exemple, par hydrogénation catalytique en présence d'un mélange de charbon et de palladium (charbon palladié) : F. Melani, L. Cecchi, G. Filacchion. J. Heterocyclic Chem.(1984) Volume 21 page 813, selon le schéma :

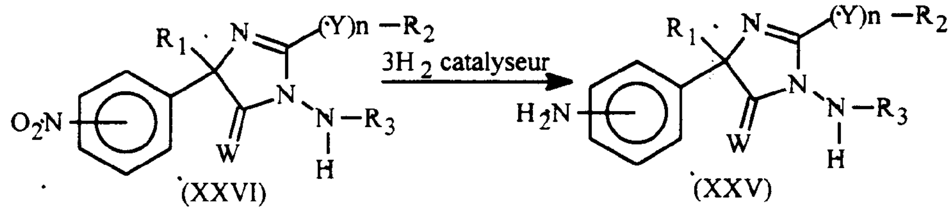


(XXVI) (XXV)

L'exemple suivant illustre ce procédé pour la préparation des composés de formule (XXV).

Exemple CI15 :

Préparation de (4-R,S)-4-(4-aminophényl)-4-méthyl-2-méthylthio-1-phénylamino-2-imidazoline-5-one, de formule développée :

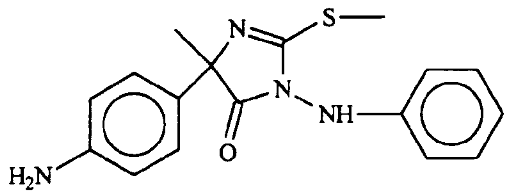

14 g (39 mmoles) de (4-R,S)-4-(4-nitrophényl)-4-méthyl-2-méthylthio-1-phénylamino-2-imidazoline-5-one, 100 ml d'acétate d'éthyle et 3 g de charbon palladié à 10% sont chargés dans un autoclave de 500 ml. L'autoclave est mis sous pression de 9 bars d'hydrogène et chauffé 12 heures à 50°C. Après filtration du catalyseur et concentration sous pression réduite, on récupère un miel jaune qui cristallise dans l'éther. Après filtration et séchage on obtient 11 g de (4-R,S)-4-(4-aminophényl)-4-méthyl-2-méthylthio-1-phénylamino-2-imidazoline-5-one sous forme d'un solide jaune fondant à 194°C, soit un rendement de 87%.

En opérant de façon analogue le (4-R,S)-4-(3-aminophényl)-4-méthyl-2-méthylthio-1-phénylamino-2-imidazoline-5-one a été obtenu avec un rendement de 95% sous forme d'un solide jaune fondant à 100°C.

Les 4-(nitrophényl)-2-imidazoline-5-ones de formule (XXVI) sont obtenues par un des procédés décrits dans la demande de brevet européen EP 0 551048 publiée le 14 Juillet 1993, dans le cas où Y=S et n=1; selon un procédé analogue au procédé C décrit précédemment dans le cas où n égale 1 et Y signifie O ; soit selon un procédé analogue au procédé D décrit précédemment lorsque n égale 0.

Procédé K:

On décrit à présent la préparation des composés de formule (Ih) :

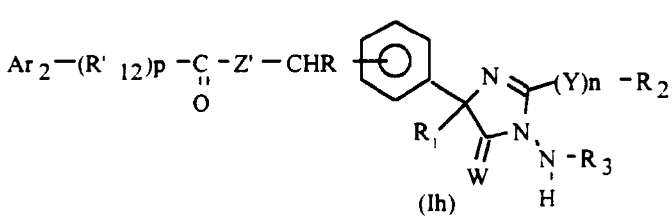

(Ih)

dans laquelle $R'^{12}$, p, Z' ont la même signification que précédemment.

Ces composés sont obtenus en faisant réagir des dérivés de type (XXVII), avec un agent électrophile de formule $Ar_2$-$(R'^{12})_p$-CO-Cl, en présence d'une base telle qu'un carbonate alcalin ou alcalinoterreux ou d'une base organique dans un solvant inerte, selon le schéma :

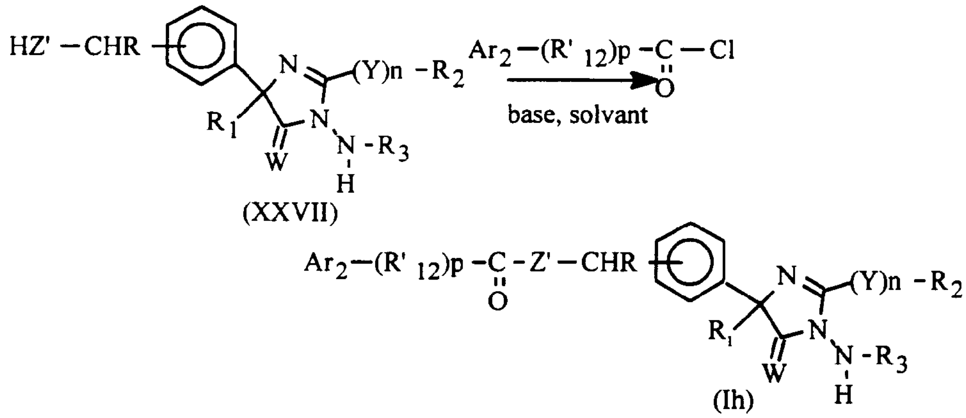


(XXVII)

(Ih)

L'exemple suivant illustre ce procédé pour la préparation des composés de formule (Ih).

Exemple CF10 :

Préparation de (4-R,S)-4-(4-(4-chlorobenzoyloxyméthyl)phényl)-4-méthyl-2-méthylthio-1-phénylamino 2-imidazoline-5-one, (composé n° 94) de formule développée :

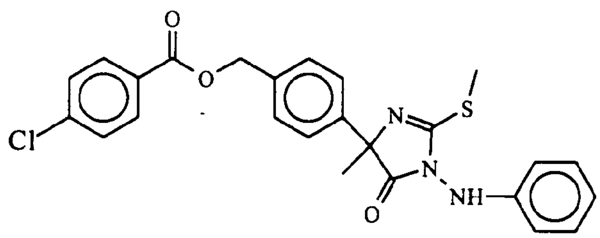

A une solution de 1g (0, 0029 mole) de (4-R,S) -4-(4-hydroxyméthylphényl)-4-méthyl-2-méthylthio-1-phénylamino 2-imidazoline-5-one dans 20 ml de THF sont ajoutés à température ambiante 0, 42 ml (0, 0029 mole) de triéthylamine puis 0, 38 ml (0, 0029 mole) de chlorure de parachlorobenzoyle. Après une demi-heure d'agitation à température ambiante, le milieu est hydrolysé puis extrait à l'acétate d'éthyle. Les extraits organiques sont lavés, séchés puis évaporés. Après mélange à l'éther isopropylique, on récupère 0, 6g de (4-R,S)-4-(4-(4-chlorobenzoyloxyméthyl)phényl)-4-méthyl-2-méthylthio-1-phénylamino 2-imidazoline-5-one sous la forme d'une poudre blanche fondant à 129°C, avec un rendement de 43%.

L'imidazolinone de formule (XXVII) peut être préparée selon un des procédés décrits précédemment, notamment par un procédé analogue à celui utilisé pour la préparation de l'imidazolinone de formule (XXII).

Procédé L:

On décrit à présent la préparation des composés de formule générale (Ii):

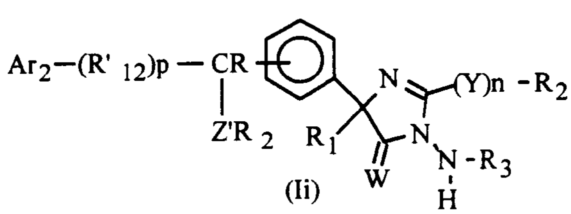

(Ii)

dans laquelle $Ar^2$, $R'^{12}$ et Z' ont la même signification que précédemment.

Ces composés sont obtenus en faisant réagir des dérivés benzyliques de 2-imidazoline-5-ones de formule (XXVIII) avec un nucléophile de formule $HZ'R^2$ éventuellement en présence d'une base telle qu'un carbonate alcalin ou alcalinoterreux ou d'une base organique selon le schéma :

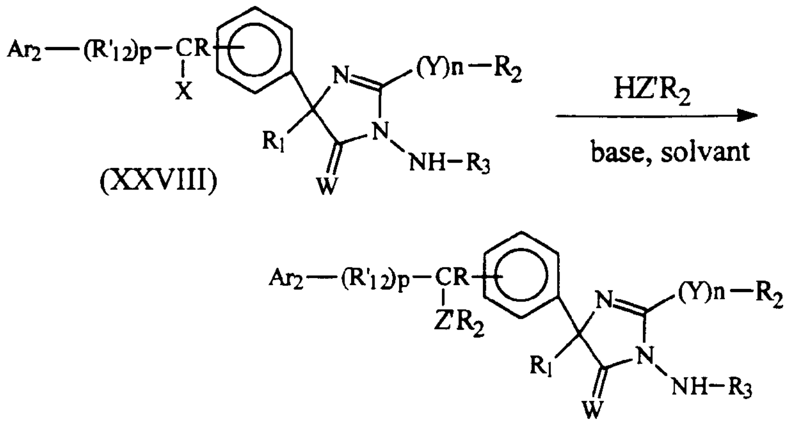


(XXVIII)

(Ii)

L'exemple suivant illustre ce procédé pour la préparation des composés de formule (Ii).

Exemple CF11 :

Préparation de (4-R,S)-4-(4-(méthoxybenzyl)phényl)-4-méthyl-2-méthylthio-1-phénylamino 2-imidazoline-5-one, (composé n° 107) de formule développée:

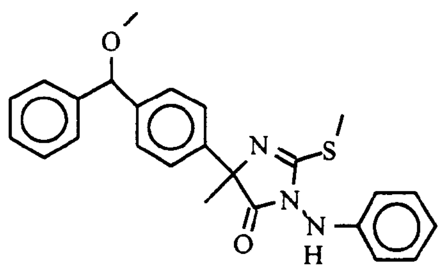

A une solution de 0, 37g (0, 007 mole) de méthylate de sodium dans 30 ml de méthanol sous atmosphère inerte on ajoute 1, 5 g (0, 0035 mole) de (4-R,S)-4-(4-(chlorobenzyl)phényl)-4-méthyl-2-méthylthio-1-phénylamino 2-imidazoline-5-one. Le mélange réactionnel est chauffé à 50°C pendant une demi-heure. Après refroidissement, le milieu est hydrolysé puis extrait à l'acétate d'éthyle. Les extraits organiques sont lavés avec une solution saturée de chlorure de sodium, séchés puis évaporés. Le produit brut ainsi obtenu est ensuite chromatographié sur colonne de silice (éluant : heptane / acétate d'éthyle 50 / 50). On récupère 0, 7 g de (4-R,S)-4-(4-(méthoxybenzyl)phényl)-4-méthyl-2-méthylthio-1-phénylamino 2-imidazoline-5-one sous la forme d'une poudre blanche fondant à 161°C avec 47% de rendement.

Les dérivés benzyliques de 2-imidazoline-5-ones de formule (XXVIII) sont préparés selon un des procédés décrits précédemment, notamment selon un procédé analogue à celui utilisé pour la préparation du composé de formule (XXIII) à partir du composé de formule (XXII).

Procédé M :

On décrit à présent la préparation des composés de formule (Ij) :

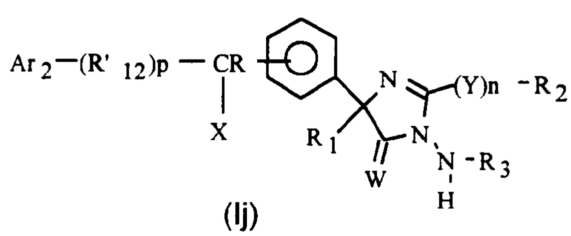

(Ij)

dans laquelle R'[12] et p ont la même signification que précédemment, Y représente l'atome de soufre, X représente un atome d'halogène, de préférence un atome de chlore.

Ces composés sont obtenus par halogénation des composés de formule (XXIX).

L'halogénation est effectuée suivant des procédés usuellement utilisés pour ce type de réaction à l'aide d'hydracide ou d'halogénure d'acide inorganique tel que $SOCl_2$, $PCl_5$, $PCl_3$, $POCl_3$ comme cité dans Advanced organic chemistry, J. March, (1985), Page 382, 3ème Edition et selon le schéma réactionnel :

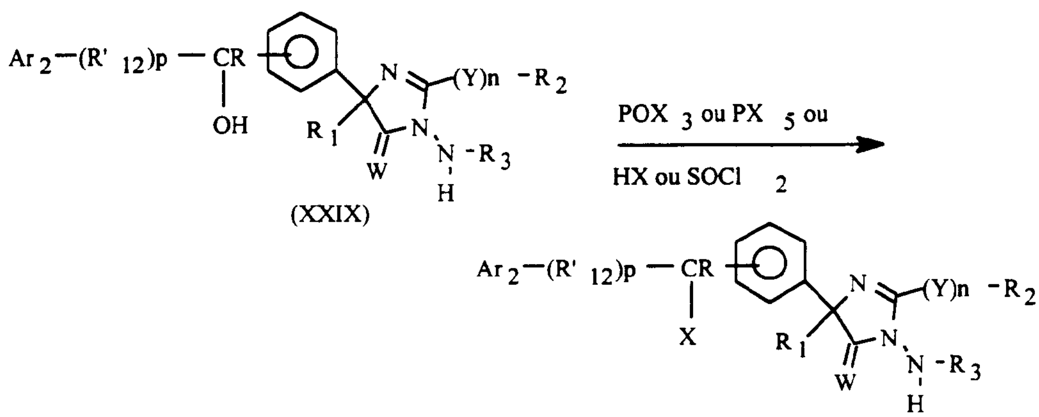


(XXIX)

(Ij)

L'exemple suivant illustre ce procédé pour la préparation des composés de formule (Ij):

Exemple CF12 :

Préparation de (4-R,S)-4-(4-(chlorobenzyl)phényl)-4-méthyl-2-méthylthio-1-phénylamino 2-imidazoline-5-one, (composé n° 106) de formule développée:

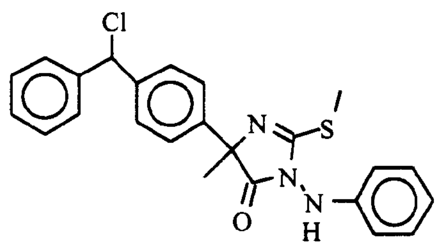

A une solution de 6, 5 g (0, 015 mole) de (4-R,S)-4-(4-((phényl) hydroxyméthyl) phényl)-4-méthyl-2-méthylthio-1-phénylamino 2-imidazoline-5-one dans 100 ml de chlorure de méthylène sont ajoutés à 0°C 1, 4 ml (0, 015 mole) de pyridine puis 1, 15 ml (0, 015 mole) de chlorure de thionyle. Le milieu réactionnel est ensuite porté au reflux pendant une demi-heure, puis refroidi jusqu'à température ambiante, hydrolysé et extrait au chlorure de méthylène. Les extraits organiques sont lavés, séchés puis évaporés. Après mélange du produit brut dans de l'éther diisopropylique, on récupère 4, 7 g de (4-R,S)-4-(4-(chlorobenzyl)phényl)-4-méthyl-2-méthylthio-1-phénylamino 2-imidazoline-5-one sous forme d'une poudre blanche fondant à 153°C avec un rendement de 69%.

Le composé de formule (XXIX) est obtenu selon un des procédés décrits précédemment, notamment selon le procédé utilisé pour préparer le composé de formule (XXII).

Les tableaux suivants rassemblent les imidazolinones de formule (I), répondant également à l'une des formules (Ik) ou (Il) indiquées ci-dessous, qui ont été préparées selon l'un des procédés qui ont été décrits précédemment (indiqué dans la colonne correspondante).

Dans ces tableaux, les radicaux méthyle, éthyle, propyle, pyridyle, pyrimidinyle, acétyle, phényle et benzyle sont respectivement représentés par Me, Et, Pr, Py, Pyrim, Ac, Ph, et Bn, et PF signifie point de fusion (PF).

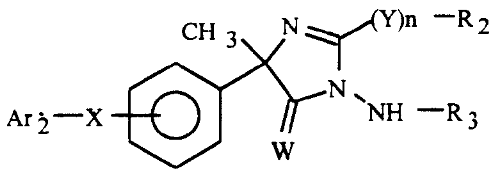

(Ik)

| N° | W | Ar2X | (Y)n R2 | R3 | PF °C | Procédé |
|---|---|---|---|---|---|---|
| 1 | O | 4-(PhC(O)NH) | SMe | Ph | 212 | J |
| 2 | O | 4-(PhC(O)NH) | OMe | Ph | 150 | C |
| 3 | O | 4-(4-Cl-PhC(O)NH) | SMe | Ph | 219 | J |
| 4 | O | 4-(4-Cl-PhC(O)NH) | OMe | Ph | 182 | C |
| 5 | O | 4-(PhNHC(O)NH) | SMe | Ph | 160 | J |
| 6 | O | 4-(PhNHC(O)NH) | OMe | Ph | 135 | C |
| 7 | O | 4-($PhSO_2NH$) | SMe | Ph | 185 | J |
| 8 | O | 3-BnO | SMe | Ph | 112 | B |

| | | | | | | |
|---|---|---|---|---|---|---|
| 9 | O | 4-BnO | OMe | Ph | 127 | C |
| 10 | O | 4-BnNH | SMe | Ph | 114 | J |
| 11 | O | 4-Bn | OMe | Ph | 148 | C |
| 12 | O | 4-Bn | SMe | Ph | 145 | B |
| 13 | O | 4-BnO | SMe | 3-F-Ph | miel | B |
| 14 | S | 4-(2-Phenethyl) | SMe | Ph | 118 | A |
| 15 | O | 4-(4-F-BnO) | SMe | Ph | 167 | G |
| 16 | O | 4-(3-F-BnO) | SMe | Ph | 133 | G |
| 17 | O | 4-(2-F-BnO) | SMe | Ph | 159 | G |
| 18 | O | 4-[(5-$CF_3$-2-Py)-O] | SMe | Ph | 163 | G |
| 19 | O | 4-(4-Me-BnO) | SMe | Ph | 176 | G |
| 20 | O | 4-(4-$CF_3$-BnO) | SMe | Ph | 170 | G |
| 21 | O | 3-(PhC(O)NH) | SMe | Ph | 130 | J |
| 22 | O | 4-(3-Me-BnO) | SMe | Ph | 151 | G |
| 23 | O | 4-(2-Me-BnO) | SMe | Ph | 146 | G |
| 24 | O | 4-[2,5-$(Me)_2$-BnO] | SMe | Ph | 149 | G |
| 25 | O | 4-PhS | SMe | Ph | 138 | B |
| 26 | O | 4-PhS | SMe | 3-F-Ph | 118 | B |
| 27 | O | 4-PhS | SMe | 3-Cl-Ph | 95 | B |
| 28 | O | 4-[$Ph(CH_2)_2$] | SMe | Ph | 155 | B |
| 29 | O | 3-(BnNH) | SMe | Ph | 72 | J |
| 30 | O | 4-[4-Cl-PhCH(OH)] | SMe | Ph | 94 | E |
| 31 | O | 4-[$PhCH_2CH(OH)$] | SMe | Ph | 83 | E |
| 32 | O | 4-(2-Py-$CH_2O$ | SMe | Ph | 139 | G |
| 33 | O | 4-[$PhCH(CH_3)O$] | SMe | Ph | 137 | G |
| 34 | O | 4-(4-Py-$CH_2O$) | SMe | Ph | 215 | G |

| | | | | | | |
|---|---|---|---|---|---|---|
| 35 | O | 4-(3-Py-$CH_2$O) | SMe | Ph | 138 | G |
| 36 | O | 4-(5-$CF_3$-2-PyO) | OMe | Ph | 88 | C |
| 37 | O | 4-(PhNHC(O)O) | SMe | Ph | 201 | G |
| 38 | O | 4-[PhS(O)] | SMe | Ph | 132 | B |
| 39 | O | 4-[PhS(O)] | SMe | 3-Cl-Ph | 164 | B |
| 40 | O | 4-[PhS(O)] | SMe | 3-F-Ph | 115 | B |
| 41 | O | 4-($PhSO_2$) | SMe | Ph | 80 | B |
| 42 | O | 4-[Ph$(CH_2)_2$] | OMe | Ph | 100 | C |
| 43 | O | 4-($PhCO_2$) | SMe | Ph | 170 | G |
| 44 | O | $PhCOCH_2O$ | SMe | Ph | 163 | G |
| 45 | O | 4-[4-Cl-PhNHC(O)] | SMe | Ph | 210 | H |
| 46 | O | 4-[PhNHC(O)] | SMe | Ph | 105 | H |
| 47 | O | 4-(2,4-diF-$PhOCH_2$) | SMe | Ph | 152 | I |
| 48 | O | 4-(3-Cl-PhNHCO) | S-Me | Ph | 115 | H |
| 49 | O | 4-(4-Cl-PhCMe(OH)) | S-Me | Ph | 90 | E |
| 50 | O | 4-(2-(4,6-diMe-Pyrim)O) | S-Me | Ph | 87 | G |
| 51 | O | 4-(2-Me-$PhCH_2$O) | S-Me | Ph | 129 | G |
| 52 | O | 4-($PhSO_2$) | S-Me | 3-Cl-Ph | 153 | B |
| 53 | O | 4-($PhSO_2$) | S-Me | 3-F-Ph | 188 | B |
| 54 | O | 4-(PhS) | Et | Ph | 85 | D |
| 56 | O | 4-($PhOCH_2$) | MeS | Ph | 153 | I |
| 57 | O | 4-(4-Cl-$PhOCH_2$) | MeS | Ph | 164 | I |
| 58 | O | 4-(3-Cl-$PhOCH_2$) | MeS | Ph | 149 | I |
| 59 | O | 4-(2-Cl-$PhOCH_2$) | MeS | Ph | 102 | I |
| 60 | O | 4-(4-F-$PhOCH_2$) | MeS | Ph | 132 | I |

| | | | | | | |
|---|---|---|---|---|---|---|
| 61 | O | 4-(3-F-$PhOCH_2$) | MeS | Ph | 147 | I |
| 62 | O | 4-(2-MeO-$PhOCH_2$) | MeS | Ph | 156 | I |
| 63 | O | 3-($PhCH_2O$) | MeO | Ph | 56 | C |
| 84 | O | 3-($PhCH_2O$) | OMe | Ph | 56 | C |
| 85 | O | 4-(2-F-$PhOCH_2$) | SMe | Ph | 153 | I |
| 86 | O | 4-PhSO | Et | Ph | miel | D |
| 87 | O | 4-$PhSO_2$ | Et | Ph | 194 | D |
| 88 | O | 4-(4-F-PhC(O)) | SMe | Ph | 163 | E |
| 89 | O | 4-(4-MeO-Ph)$OCH_2$ | SMe | Ph | 151 | I |
| 90 | O | 4-(3-MeO-Ph)$OCH_2$ | SMe | Ph | 131 | I |
| 91 | O | 4-(4-$CF_3$-Ph)$OCH_2$ | SMe | Ph | 173 | I |
| 92 | O | 4-(3-MeO-$PhCH_2O$) | SMe | Ph | 126 | G |
| 93 | O | 4-(2-F-$PhOCH_2$) | OMe | Ph | 165 | C |
| 94 | O | 4-(4-Cl-$PhCO_2CH_2$) | SMe | Ph | 129 | K |
| 95 | O | 4-PhSO | OMe | Ph | miel | C |
| 96 | O | 4-(4-Cl-Ph)$CH_2O$ | SMe | Ph | 187 | G |
| 98 | O | 4-PhCH(OH) | SMe | Ph | miel | E |
| 99 | O | 4-(4-MeO-$PhCH_2O$) | SMe | Ph | 156 | G |
| 100 | O | 4-(4-NC-$PhCH_2O$) | SMe | Ph | 209 | G |
| 101 | O | 4-$PhOCH_2$ | OMe | Ph | 158 | C |
| 102 | O | 4-(3-Cl-$PhOCH_2$) | OMe | Ph | 147 | C |
| 103 | O | 4-(3-F-$PhOCH_2$) | OMe | Ph | 179 | C |
| 104 | O | 4-(4-F-$PhOCH_2$) | OMe | Ph | 141 | C |
| 105 | O | 4-(4-$CF_3$-$PhOCH_2$) | OMe | Ph | 143 | C |
| 106 | O | 4-PhCH(Cl) | SMe | Ph | 153 | M |
| 107 | O | 4-PhCH(OMe) | SMe | Ph | 161 | L |

| | | | | | | |
|---|---|---|---|---|---|---|
| 108 | O | 4-PhCH(OMe) | OMe | Ph | 136 | C |
| 109 | O | 4-PhS | SMe | 2,5-diF-Ph | 104 | B |
| 110 | O | 4-PhCO | SMe | Ph | 162 | E |
| 111 | O | 4-(2-CN-$PhCH_2O$) | SMe | Ph | 174 | G |
| 112 | O | 4-PhCO | OMe | Ph | 83 | C |
| 113 | O | 3-(4-Cl-$PhCOOCH_2$) | SMe | Ph | 156 | K |
| 114 | O | 3-(4-Cl-$PhCOOCH_2$) | OMe | Ph | 105 | C |
| 115 | O | 4-PhS | SMe | 2,3-diF-Ph | 113 | B |
| 116 | O | 4-(3-CN-$PhCH_2O$) | SMe | Ph | 171 | G |
| 117 | O | 3-(4-CF3-$PhOCH_2$) | SMe | Ph | 127 | I |
| 118 | O | 3-(PhCO) | SMe | Ph | 149 | E |
| 119 | O | 3-(4-Cl-PhCH(OH)) | SMe | Ph | 156 | E |
| 120 | O | 3-(4-Cl-$PhOCH_2$) | SMe | Ph | 112 | I |
| 121 | O | 3-(4-$CF_3$-$PhOCH_2$) | OMe | Ph | 102 | C |
| 122 | O | 3-(PhCH(OMe)) | OMe | Ph | 120 | C |
| 123 | O | 3-(4-Cl-PhCH(OMe)) | OMe | Ph | 120 | C |
| 124 | O | 3-(4-Cl-$PhOCH_2$) | OMe | Ph | miel | C |
| 125 | O | 3-(4-Me-$PhOCH_2$) | SMe | Ph | 87 | I |
| 126 | O | 3-(4-F-$PhOCH_2$) | OMe | Ph | 136 | C |
| 127 | O | 3-(4-F-PhCH(OH)) | SMe | Ph | 128 | E |
| 128 | O | 3-(4-$CF_3$-PhCH(OH)) | SMe | Ph | 145 | E |
| 129 | O | 3-(4-OMe-PhCH(OH)) | SMe | Ph | 114 | E |
| 130 | O | 3-(4-F-PhCH(OMe)) | OMe | Ph | 131 | C |
| 131 | O | 3-($PhCH_2O$) | SMe | Ph | 132 | I |
| 132 | O | 3-(PhCO) | SMe | Ph | miel | E |
| 133 | O | 3-($PhOCH_2$) | OMe | Ph | miel | C |

| | | | | | | |
|---|---|---|---|---|---|---|
| 134 | O | 4-(4-FPh$CH_2$O) | OMe | Ph | 171 | G |
| 135 | O | 4-[(2-Benzothiazolyl)O] | SMe | Ph | 144 | G |

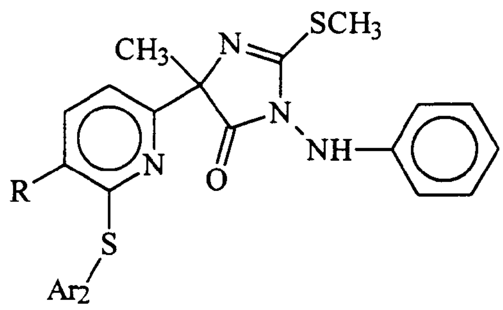

(II)

| N° | Ar2 | R | PF °C | Procédé |
|---|---|---|---|---|
| 64 | 4-F-Ph | $NO_2$ | 171 | F |
| 65 | Ph | $NO_2$ | 148 | F |
| 66 | 2-F-Ph | $NO_2$ | 115 | F |
| 67 | 3-F-Ph | $NO_2$ | 112 | F |
| 68 | 4-Cl-Ph | $NO_2$ | 146 | F |
| 69 | 3-Cl-Ph | $NO_2$ | 103 | F |
| 70 | 2-Cl-Ph | $NO_2$ | 93 | F |
| 71 | 2-Naphtyl | $NO_2$ | 131 | F |
| 72 | 2-OH-Ph | $NO_2$ | 97 | F |
| 73 | 4-HOPh | $NO_2$ | 145 | F |
| 74 | 4-MeOPh | $NO_2$ | 134 | F |
| 75 | 3-MeOPh | $NO_2$ | 165 | F |

| | | | | |
|---|---|---|---|---|
| 76 | 4-MePh | $NO_2$ | 115 | F |
| 77 | 2-MePh | $NO_2$ | 120 | F |
| 78 | 3-MePh | $NO_2$ | 113 | F |
| 79 | Ph | $CF_3$ | 166 | F |
| 80 | 4-Me-Ph | $CF_3$ | 149 | F |
| 81 | 4-MeO-Ph | $CF_3$ | 151 | F |
| 82 | 4-Cl-Ph | $CF_3$ | 149 | F |
| 83 | 3-F-Ph | $CF_3$ | 143 | F |

La présente invention a pour objet certains des nouveaux composés décrits précédemment comme intermédiaires, à savoir les composés de formules IIIa, IIIb, IV, VII, XIII, XIX, XX, XXII, XXIV, et XXVII dans lesquelles les substituants ont la signification définie précédemment. Comme les composés définis par la formule générale (I) de l'invention, ces composés peuvent exister sous une ou plusieurs formes isomérique selon le nombre de centres asymétriques de la molécule. L'invention concerne donc aussi bien tous les isomères optiques des composés utilisables commme intermédiaires que leurs mélanges racémiques et les diastéréoisomères correspondants, séparés ou en mélange, et notamment les énantiomères des composés comprenant le carbone asymétrique porteur de $R_1$. La séparation des isomères optiques et/ou des diastéréoisomères à partir des mélanges racémiques peut s'effectuer selon des méthodes connues en soi. Les énantiomères des composés intermédiaires comprenant le carbone asymétrique porteur de $R_1$ peuvent également être préparées, selon des procédés connus en soi ou selon les procédés décrits précédemment, à partir des matières premières chirales, et notamment à partir des $\alpha$ amino acides chiraux de formule (IX), c'est-à-dire à partir des composés de formule (IX) dans laquelle le carbone rattaché à $R_1$ est un carbone asymétrique.

L'invention concerne également un procédé de traitement des plantes cultivées atteintes ou susceptibles d'être atteintes par les maladies fongiques caractérisé en ce que l'on applique sur les parties aériennes de ces plantes une dose efficace d'un composé selon la formule (I). Par dose efficace, on entend une quantité suffisante pour permettre le contrôle et la destruction des champignons présents sur ces plantes cultivées. Les doses d'utilisation peuvent toutefois varier dans de larges limites selon le champignon à combattre, le type de culture, les conditions climatiques, et selon le composé utilisé.

En pratique, les composés s'appliquent avantageusement à des doses de 0,002 à 5 kg/ha, et de préférence de 0,005 à 1 kg/ha.

Par maladies fongiques, on entend les maladies causées par les champignons phytopathogènes, notamment ceux de la famille des oomycètes, des ascomycètes et des basidiomycètes.

Parmi les cultures pouvant faire l'objet d'un traitement fongicide à l'aide d'un composé selon l'invention, on peut citer le riz, les céréales, notamment le blé et l'orge, ainsi que les plantes légumières. Le riz est une culture préférée pour les traitements fongicides à l'aide d'un composé selon l'invention.

Les exemples qui suivent illustrent la bonne activité fongicide des composés selon l'invention.

**Exemple B1** :

Test in vivo sur *Pyricularia oryzae* responsable de la piriculariose du riz :

On prépare, par broyage fin, une suspension aqueuse de la matière active à tester ayant la composition suivante :

- matière active : 60 mg
- acétone : 5 ml
- agent tensioactif (oléate de dérivé polyoxyéthyléné du sorbitan) dilué à 10% dans l'eau : 0,3 ml
- on complète à 60 ml d'eau.

Cette suspension aqueuse est ensuite diluée par de l'eau pour obtenir la concentration désirée en matière active.

Du riz, semé en godets dans un mélange 50/50 de tourbe enrichie et de pouzzolane, est traité au stade d'environ 10 cm de hauteur ( ce qui correspond au stade 2-3 feuilles) par pulvérisation de la suspension aqueuse ci dessus.

Au bout de 24 heures, on applique sur les feuilles une suspension aqueuse de spores de Pyricularia oryzae, obtenue à partir d'une culture de 15 jours, mise ensuite en suspension à raison de 100 000 unités par $cm^3$.

Les plants de riz sont placés pendant 24 heures en incubation ( 25°C, 100% d'humidité relative) , puis mis en cellule d'observation, dans les mêmes conditions, pendant 5 jours.

La lecture se fait 6 jours après la contamination.

Dans ces conditions, on observe, à la dose de 1 g/l, une protection bonne (au moins 75%) ou totale avec les composés suivants : 9, 11, 15, 16, 23, 27 à 29, 31 à 33, 35 à 37, 42, 67, 68, 84, 92, 94, 95, 96, 102, 104, 105, 108, 109, 112, 114, 121.

**Exemple B2** :

Test in vivo sur *Puccinia recondita* responsable de la rouille brune du blé :

On prépare, par broyage fin, une suspension aqueuse de la matière active à tester ayant la composition suivante:

- matière active: 60 mg
- acétone : 5 ml
- agent tensioactif Tween 80, (oléate de dérivé polyoxyéthyléné du sorbitan) dilué à 10% dans l'eau : 0,3 ml
- on complète à 60 ml d'eau.

Cette suspension aqueuse est ensuite diluée par de l'eau pour obtenir la concentration désirée en matière active.

Du blé semé sur un substrat tourbe terre pouzzolane 50/50 dans un godet et maintenu à 12 °C, est traité au stade 10 cm de hauteur par pulvérisation de la suspension aqueuse ci dessus.

Au bout de 24 heures, une suspension aqueuse de spores (100 000 sp/$cm^3$) est pulvérisée sur le blé; cette suspension a été obtenue à partir de plants contaminés. On place ensuite le blé pendant 24 heures en cellule d'incubation à environ 20°C et à 100% d'humidité relative, puis pendant 7 à 14 jours à 60% d'humidité relative.

Le contrôle de l'état des plants se fait entre le 8ème et le 15ème jour après la contamination, par comparaison avec un témoin non traité.

Dans ces conditions, on observe, à la dose de 1 g/l, une protection bonne (au moins 75%) ou totale avec les composés suivants : 4, 8 à 13, 15 à 18, 20 à 28, 31 à 33, 35, 36, 38, 40 à 43, 48, 49, 73, 79, 81, 84, 85, 89, 90, 93, 94, 95, 96, 98, 99, 100, 101, 102, 103, 104, 105, 106, 107, 108, 109, 110, 115, 116, 117, 119, 120, 121.

**Exemple B3** :

Test in vivo sur *Septoria tritici* responsable de la septoriose du blé :

Une suspension aqueuse, de concentration 1 g/l, de la matière active testée est obtenue par broyage de 60 mg de celle-ci dans le mélange suivant :

- acétone : 5 ml

agent tensioactif (oléate de dérivé polyoxyéthyléné du sorbitan) dilué à 10 % : 0,3 ml

puis le volume est ajusté à 60 ml avec de l'eau.

Cette suspension aqueuse est ensuite diluée par de l'eau pour obtenir la concentration désirée en matière active.

Des plants de blé (variété Darius), semés sur un substrat tourbe-pouzzolane 50/50 et cultivés en serre à température 10-12 °C, sont traités au stade 1 feuille (taille de 10 cm environ) par pulvérisation de la suspension de matière active décrite ci-dessus.

Des plants, utilisés comme témoins, sont traités par pulvérisation d'une solution aqueuse ne contenant pas la matière active.

24 heures après traitement, les plants sont contaminés par pulvérisation d'une suspension aqueuse de spores (500 OOOsp/ml) récoltés sur une culture âgée de 7 jours.

Après contamination, les plants sont placés à 18°C en atmosphère humide. La notation est effectuée 20 jours après la contamination en comparaison avec les plants témoins.

Dans ces conditions, on observe à la dose de 1 g/l, une bonne protection (au moins 75 % ) ou totale avec les composés : 9, 15, 21, 23, 32, 36,49, 73,88, 89, 90, 93, 94, 95, 98, 104, 105, 108, 112, 114, 117, 118, 120,

121.

La présente invention a également pour objet des compositions, utilisables comme agents fongicides, contenant comme matière(s) active(s) un (ou plusieurs) composé selon la formule (I) tel que décrit précédemment, en mélange avec les supports solides ou liquides, acceptables en agriculture et les agents tensio-actifs également acceptables en agriculture. En particulier sont utilisables les supports inertes et usuels et les agents tensio-actifs usuels.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... Plus généralement les composés utilisés dans l'invention peuvent être combinés à tous les additifs solides ou liquides correspondant aux techniques habituelles de la mise en formulation.

D'une façon générale, les compositions selon l'invention contiennent habituellement de 0,05 à 95 % environ (en poids) d'un composé selon l'invention (appelé par la suite matière active), un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, notamment le butanol etc...).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque le composé et/ou le support inerte ne sont pas solubles dans l'eau et que l'agent vecteur de l'application est l'eau.

Ainsi donc, les compositions à usage agricole selon l'invention peuvent contenir les matières actives selon l'invention dans de très larges limites, allant de 0,05 % à 95 % (en poids). Leur teneur en agent tensio-actif est avantageusement comprise entre 5 % et 40 % en poids.

Ces compositions selon l'invention sont elles-mêmes sous des formes assez diverses, solides ou liquides.

Comme formes de compositions solides, on peut citer les poudres pour poudrage (à teneur en composé pouvant aller jusqu'à 100 %) et les granulés, notamment ceux obtenus par extrusion, par compactage, par imprégnation d'un support granulé, par granulation à partir d'une poudre (la teneur en composé dans ces granulés étant entre 0,5 et 80 % pour ces derniers cas), les comprimés ou tablettes effervescents.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes, les gels.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures.

A titre d'exemple, voici la composition de quelques concentrés émulsionnables :

Exemple CE 1 :

- matière active 400 g/l
- dodécylbenzène sulfonate alcalin 24 g/l

- nonylphénol oxyéthylé à 10 molécules d'oxyde d'éthylène 16 g/l
- cyclohexanone 200 g/l
- solvant aromatique q.s.p.1 litre

Selon une autre formule de concentré émulsionnable, on utilise :

Exemple CE 2

- matière active 250 g
- huile végétale époxydée 25 g
- mélange de sulfonate d'alcoylaryle et d'éther de polyglycol et d'alcools gras 100 g
- diméthylformamide 50 g
- xylène 575 g

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

Exemple SC 1 :

- matière active 500 g
- phosphate de tristyrylphénol polyéthoxylé 50 g
- alkylphénol polyéthoxylé 50 g
- polycarboxylate de sodium 20 g
- éthylène glycol 50 g
- huile organopolysiloxanique (antimousse) 1 g
- polysaccharide 1,5 g
- eau 316,5 g

Les poudres mouillables (ou poudre à pulvériser) sont habituellement préparées de manière qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0,1 à 10 % d'un ou plusieurs stabilisants et/ou autres additifs, comme des agents de pénétration, des adhésifs, ou des agents antimottants, colorants, etc...

Pour obtenir les poudres à pulvériser ou poudres mouillables, on mélange intimement les matières actives dans les mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables trés avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

Exemple PM 1

- matière active 50%
- alcool gras éthoxylé (agent mouillant) 2,5%
- phényléthylphénol éthoxylé (agent dispersant) 5%
- craie (support inerte) 42,5%

Exemple PM 2 :

- matière active 10%
- alcool synthétique oxo de type ramifié, en C13 éthoxylé par 8 à 10 oxyde d'éthylène (agent mouillant) 0,75%
- lignosulfonate de calcium neutre (agent dispersant) 12%

- carbonate de calcium (charge inerte) q.s.p. 100 %

Exemple PM 3 :

Cette poudre mouillable contient les mêmes ingrédients que dans l'exemple précédent, dans les proportions ci-après :

- matière active 75 %
- agent mouillant 1,50%
- agent dispersant 8%
- carbonate de calcium (charge inerte) q.s.p. 100%

Exemple PM 4 :

- matière active 90%
- alcool gras éthoxylé (agent mouillant) 4%
- phényléthylphénol éthoxylé (agent dispersant) 6%

Exemple PM 5 :

- matière active 50%
- mélange de tensio-actifs anioniques et non ioniques (agent mouillant) 2,5%
- lignosulfonate de sodium (agent dispersant) 5%
- argile kaolinique (support inerte) 42,5%

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les composés selon l'invention peuvent être formulés sous la forme de granulés dispersibles dans l'eau également compris dans le cadre de l'invention.

Ces granulés dispersibles, de densité apparente généralement comprise entre environ 0,3 et 0,6 ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnées ci-dessus. On peut encore utilisé des granulés obtenus comme précédemment puis imprégnés avec une composition contenant la matière active.

De préférence, il est obtenu par extrusion, en opérant comme indiqué dans les exemples ci-après.

Exemple GD1 : Granulés dispersibles

Dans un mélangeur, on mélange 90 % en poids de matière active et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé

qui est séché, puis concassé et tamisé, de façon à ne garder respectivement que les granulés d'une dimension comprise entre 150 et 2000 microns.

<u>Exemple GD2</u> : <u>Granulés dispersibles</u>

Dans un mélangeur, on mélange les constituants suivants :

- matière active 75%
- agent mouillant (alkylnaphtalène sulfonate de sodium) 2%
- agent dispersant (polynaphtalène sulfonate de sodium) 8%
- charge inerte insoluble dans l'eau (kaolin) 15%

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

En ce qui concerne les compositions adaptées au stockage et au transport, elles contiennent plus avantageusement de 0,5 à 95 % (en poids) de substance active.

## Revendications

1. Composés dérivés de 2-imidazoline-5-ones, caractérisés en ce qu'ils répondent à la formule générale (I) :

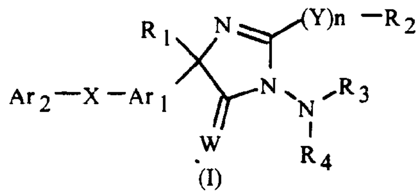

(I)

dans laquelle :

- $R_1$ représente un atome d'hydrogène, ou un groupe vinyle ou allyle, ou un radical alkyle ou haloalkyle, chacun de 1 à 3 atomes de carbone ;
  ou encore, $R_1$ et $Ar_2$-X-$Ar_1$ peuvent en outre former, avec le carbone auquel ils sont liés sur le cycle imidazolinone. le motif suivant :

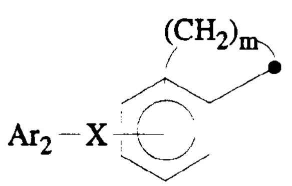

dans lequel le point en caractère gras représente le carbone du cycle imidazolinone de la formule (I) auquel sont liés les radicaux $R_1$ et $Ar_2$-X-$Ar_1$, et m égale 2, 3 ou 4 ;

- n = 0 ou 1 ;
- Y représente l'atome d'oxygène ou de soufre ;
- $R_2$ représente :
  - l'atome d'hydrogène quand n égale 0 ou
  - un groupe alkyle ou haloalkyle, chacun de 1 à 3 atomes de carbone ou le groupe cyclopropyle ;
- $R_3$ représente un radical aryle ou hétéroaryle comprenant phényle, naphtyle, pyridyle, pyrimidyle, pyridazinyle, pyrazinyle, thiazolyle, benzothiényle, benzofuryle, quinolinyle, isoquinolinyle, benzothiazolyle ou méthylène dioxyphényle, chacun de ces radicaux étant éventuellement substitué par 1 à 7 groupements, de préférence de 1 à 3 groupements, choisis parmi les significations de $R_5$ défini ci-après ;
- $R_4$ représente un atome d'hydrogène, un radical formyle ou aroyle, un radical acyle de 2 à 6 atomes

de carbone, un radical alkoxycarbonyle de 2 à 6 atomes de carbone, un radical aryloxycarbonyle ou arylsulfonyle, un radical alkylsulfonyle de 1 à 6 atomes de carbone, ou un radical alkyloxalyle ou alkoxyoxalyle de 3 à 6 atomes de carbone ;

- $R_5$ représente:
  - un atome d'halogène ou le groupe hydroxy, mercapto, nitro, $SF_5$, cyano, thiocyanato ou azido; ou
  - un radical alkyle, haloalkyle, cyanoalkyle, alkoxy, haloalkoxy, cyanoalkoxy, alkylthio, haloalkylthio, cyanoalkylthio, alkylsulfinyle, alkylsulfonyle, haloalkylsulfinyle ou haloalkylsulfonyle, chacun de 1 à 6 atomes de carbone ; ou
  - un radical cycloalkyle, halocycloalkyle, alcényle, alcynyle, alcényloxy, alcynyloxy, alcénylthio, alcynylthio, chacun de 3 à 6 atomes de carbone ; ou
  - un radical amino éventuellement mono ou disubstitué par un radical alkyle ou acyle de 1 à 6 atomes de carbone ou alkoxycarbonyle de 2 à 6 atomes de carbone ;ou
  - un groupe alkoxycarbonyle de 2 à 7 atomes de carbone ; ou
  - un groupe N-alkylcarbamoyle de 2 à 7 atomes de carbone ; ou
  - un groupe N,N-dialkylcarbamoyle de 3 à 13 atomes de carbone ;
- W représente O, S ou SO ;
- $Ar_1$ est un radical divalent qui dérive d'un radical aryle ou hétéroaryle comprenant les radicaux phényle, naphtyle, thiényle, furyle, pyrrolyle, pyridyle, benzothiényle, benzofuryle, indolyle, quinolinyle, isoquinolinyle, ou méthylènedioxyphényle, chacun de ces radicaux étant éventuellement substitué par 1 à 6 groupements, de préférence par 1 à 3 groupements, choisis parmi les significations de $R_5$ ;
- $Ar_2$ représente un système mono ou bicyclique de 5 à 10 atomes, qui est aromatique, saturé ou insaturé, et qui est soit un système carbocyclique, soit un système hétérocyclique contenant de 1 à 4 hétéroatomes choisis parmi O, S, ou N, et qui comprend les radicaux phényle, naphtyle, dihydronaphtyle, tétrahydronaphtyle, thiényle, furyle, pyrrolyle, pyridyle, benzothiényle, benzofuryle, indanyle, indolyle, quinolinyle, isoquinolinyle, méthylènedioxyphényle, imidazolyle, pyrazolyle, triazolyle, oxazolyle, isoxazolyle, thiazolyle, isothiazolyle, oxadiazolyle, thiadiazolyle, benzimidazolyle, indazolyle, benzoxazolyle, benzisoxazolyle, benzothiazolyle, benzisothiazolyle, pyrimidyle, pyrazinyle, pyridazinyle,triazinyle, naphtyridyle, quinoxazolyle, quinazolyle, cinnolyle ou phtalazinyle, chacun de ces radicaux étant éventuellement substitué par par 1 à 7 groupements, de préférence par 1 à 3 groupements, choisis parmi les significations de $R_5$ ;
- X représente un enchaînement de formule générale :

$$-(R'')_j-A-(R')_k-$$

dans lequel :
  - j et k, identiques ou différents, sont égaux à 0 ou 1 ;
  - R"et R', identiques ou différents, représentent une chaîne hydrocarbonée, saturée ou insaturée, qui contient de 1 à 6 atomes de carbone, éventuellement substituée par 1 à 12 groupements, de préférence par 1 à 3 groupements, choisis parmi $R_7$ défini ci-après ; et dans laquelle un ou plusieurs atomes de carbone peuvent être remplacés par O, S, $N(R_5)$, $R_5$ étant défini ci-après ;
  - A représente O, S, $N(R_6)$, SO, $SO_2$, CO, CS, , $Si(R_8)(R'_8)$, $N_2$, $C(R_{10})(R'_{10})$, ou -C≡C- ou

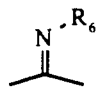 ou 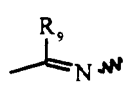 ou 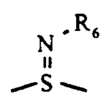 ou 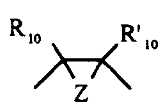

ou

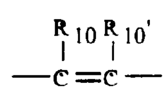

dans lequel Z représente O,S, $C(R_{11})(R'_{11})$ ; $R_8$ à $R_{11}$ étant définis ci-après ;

- $R_6$ représente :
  - un atome d'hydrogène ou un groupe cyano, hydroxy, amino, formyle, morpholino, pipéridino, pyrrolidino, pipérazino, ou
  - un radical alkyle, haloalkyle, cyanoalkyle, alkoxy, haloalkoxy, cyanoalkoxy, alkylsulfonyle, haloalkylsulfonyle, cyanoalkylsulfonyle, chacun comprenant de 1 à 6 atomes de carbone, ou
- un radical monoalkylamino ou dialkylamino, ou
- un radical cycloalkyle, halocycloalkyle, alcényle, alcynyle, alcényloxy, alcynyloxy, chacun de 3 à 6 atomes de carbone ; ou
- un radical acyle ou alkoxycarbonyle, chacun de 2 à 6 atomes de carbone ; ou
- un radical carbamoyle (éventuellement substitué par un ou deux radicaux alkyle) de 1 à 7 atomes de carbone ou sulfamoyle de 1 à 6 atomes de carbone ; ou
- un radical aroyle ou arylsulfonyle ; ou
- un radical alkyloxalyle ou alkoxyoxalyle, chacun de 3 à 8 atomes de carbone ; ou
- un radical oxamyle (éventuellement substitué par un ou deux radicaux alkyle) de 2 à 8 atomes de carbone

- $R_7$ représente un atome d'halogène, un groupe cyano, thiocyanato, hydroxycarbonyl, amino (éventuellement substitué), hydroxyle, oxo, alkoxy, alkoxycarbonyl, haloalkoxy, alkoxyalkoxy, thiol, alkylthio, haloallcylthio, alkoxyalkylthio, acyloxy, alkyle, haloalkyle, alkoxyalkyle, alkylidène, aroyloxy, hétéroaroyloxy, arylacyloxy, cycloalkylcarbonyloxy, acylthio, aroylthio, hétéroaroylthio, arylacylthio, cycloalkylcarbonylthio, carbamoyloxy (éventuellement substitué), carbamoylthio (éventuellement substitué), thiocarbamoyloxy (éventuellement substitué), dithiocarbamoyle (éventuellement substitué), acylamino, cycloalkylcarbonylamino, aroylamino, uréïdo (éventuellement substitué), thiouréïdo,alkoxycarbonylamino, aryloxycarbonylamino, alkylsulfonylamino, arylsulfonylamino, aminosulfonylamino (éventuellement substitué), étant entendu que:
  - par éventuellement substitué on entend des radicaux éventuellement substitués par un ou deux radicaux alkyles,
  - chaque radical hydrocarboné aliphatique a de 1 à 4 atomes de carbone, et ;
  - le radical cycloalkyle contient de 3 à 7 atomes de carbone ;
- $R_8$ et $R'_8$, identiques ou différents, représentent :
  - un radical alkyle de 1 à 6 atomes de carbone ; ou
  - un radical cycloalkyle de 3 à 7 atomes de carbone ; ou
  - un radical alcényle, alcynyle de 2 à 6 atomes de carbone ; ou
  - un radical arylalkyle, de préférence benzyle, ou un radical aryle éventuellement substitué, de préférence phényle éventuellement substitué par 1 à 5 groupements, de préférence par 1 à 3 groupements, choisis parmi les significations de $R_5$ ;
- $R_9$ représente :
  - l'atome d'hydrogène, ou un groupe morpholino, pipéridino, pyrrolidino, pipérazino ; ou
  - un radical alkyle, haloalkyle, cyanoalkyle, alkoxy, haloalkoxy, cyanoalkoxy, alkylthio, haloalkylthio, cyanoalkylthio, chacun de 1 à 6 atomes de carbone ou dialkylamino contenant de 2 à 6 atomes de carbone ;
  - $R_{10}$ et $R'_{10}$, identiques ou différents, représentent l'atome d'hydrogène ou un groupe alkoxy contenant 1 à 6 atomes de carbone ou $R_7$ ;
  - $R_{11}$ et $R'_{11}$, identiques ou différents, représentent l'atome d'hydrogène ou un halogène ou un groupe alkyle de 1 à 3 atomes de carbone ;

ainsi que les formes salifiées, et les énantiomères et stéréoisomères de ces composés, notamment les 2 énantiomères de chacun de ces composés correspondant au carbone asymétrique du cycle imidazolinone porteur de $R_1$ ;

et à l'exception des composés pour lesquels :
- lorsque $(Y)_n$-$R_2$ représente un méthylthio, un méthyle ou un éthyle, $Ar_2$-X-$Ar_1$ représente un groupe phénoxyphényle, éventuellement substitué ;
- lorsque $(Y)_n$-$R_2$ représente un méthoxy, lorsque $R_1$ est un méthyle, lorsque $R_3$ représente un radical phényle ou pyridyle, éventuellement substitué par un atome de fluor ou un groupe méthyle, lorsque $R_4$ est un hydrogène, et lorsque W représente un atome d'oxygène, alors $Ar_2$-X-$Ar_1$ représente soit un groupe 4-phénoxyphényle, éventuellement substitué sur sa partie phénoxy par 1 ou 2 atomes de fluor, soit le groupe 3-phénoxyphényle ;

et à l'exception du composé de formule :

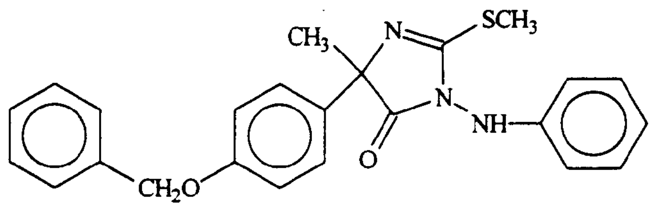

2. Composés selon la revendication 1, caractérisés en ce que :
   - $Ar_1$ représente un radical divalent dérivé d'un phényle, naphtyle, thiényle, pyridyle, benzothiényle, quinolinyle, chacun de ces radicaux étant éventuellement substitué par 1 à 3 groupements, choisis parmi les significations de $R_5$ ;
   - $Ar_2$ représente un radical phényle, naphtyle, thiényle, pyridyle, benzothiényle, thiazolyle, benzothiazolyle, pyrimidyle, chacun de ces radicaux étant éventuellement substitué par 1 à 3 groupements, choisis parmi les significations de $R_5$.

3. Composés selon l'une des revendications 1 ou 2 caractérisés en ce que :
   - $Ar_1$ représente un radical divalent dérivé d'un phényle, thiényle, pyridyle, éventuellement substitué par 1 à 3 groupements choisis parmi un atome d'halogène, un radical alkyle ou haloalkyle ;
   - $Ar_2$ représente un radical phényle, naphtyle, pyridyle, benzothiazolyle, éventuellement substitué par 1 à 3 groupements choisis parmi un atome d'halogène, un radical alkyle ou haloalkyle ;
   - X représente l'enchaînement de formule générale :

$$-(R")_j-A-(R')_k-$$

   dans laquelle :
   - R" et R', identiques ou différents, représentent une chaîne carbonée de 1 à 3 atomes de carbone, de préférence un groupe méthylène, dans laquelle un atome de carbone est éventuellement remplacé par O ou NH, et qui est éventuellement substituée par 1 à 3 groupements choisis parmi un atome d'halogène, un groupe hydroxyle, oxo ou un radical $C_1$-$C_3$ alkyle ;
   - A est O, S, SO, $SO_2$, CO, $C(R_{10})(R'_{10})$,

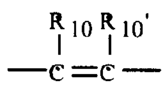

ou

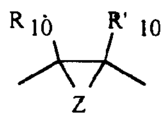

   dans lesquels :
   - $R_{10}$ et $R'_{10}$, identiques ou différents, sont un atome d'hydrogène, d'halogène, un radical hydroxyle, $C_1$-$C_3$ alkyle ou $C_1$-$C_3$ alkoxy ;
   - Z représente O ou $C(R_{11})(R'_{11})$, où $R_{11}$ et $R'_{11}$, identiques ou différents, sont un atome d'hydrogène ou de chlore.

4. Composés selon l'une des revendications 1 à 3, caractérisés en ce qu'ils répondent à la formule I dans laquelle simultanément :
   - $R_1$ est un radical méthyle ;
   - $R_2$ est un radical méthyle ou éthyle ;
   - $R_3$ représente un radical phényle ou pyridyle ;
   - $R_4$ est un atome d'hydrogène ou un radical formyle ;
   - $R_5$ est un atome d'halogène ou un radical $C_1$-$C_3$ alkyle ou $C_1$-$C_3$ haloalkyle ; et
   - W est un atome d'oxygène.

5. Procédé de préparation des composés de formule (I) selon la revendication 1, dans laquelle $R_4$ est différent de l'atome d'hydrogène, par réaction des composés de formule Ia avec un composé de formule $R_4L'$, en présence d'une base et en milieu solvant, selon le schéma :

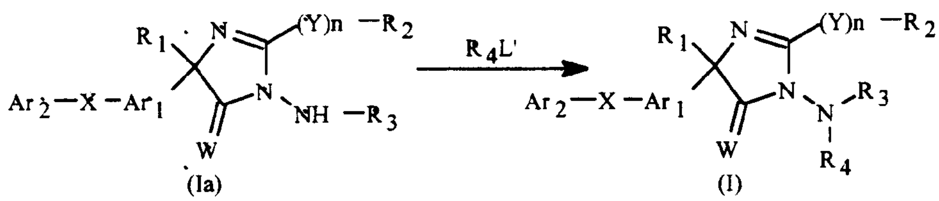


(Ia) (I)

dans lequel :

- $R_4$ représente le radical formyle, un groupe acyle de 2 à 6 atomes de carbone, aroyle, alkoxycarbonyle de 2 à 6 atomes de carbone, aryloxycarbonyle, alkylsulfonyle, arylsulfonyle, ou un radical alkyloxalyle ou alkoxyoxalyle de 3 à 6 aomes de carbone ;
- L' représente :

  un atome d'halogène, de préférence chlore, brome ou iode, le groupe sulfate,
  un radical aryloxy ou arylthio, éventuellement substitué, de préférence phénoxy,
  un radical alkoxy ou dialkylamino,
  un groupe $R_4O$ lorsque $R_4$ est un groupe acyle ou arylsulfonyl ou alkylsulfonyl.

**6.** Procédé selon la revendication 5, caractérisé en ce que on utilise comme base une base forte telle qu'un hydrure ou hydroxyde alcalin ou alcalinoterreux, un alcoolate ou une amine tertiaire, en ce que la réaction est pratiquée à une température comprise entre -30°C et + 50°C et en ce que le solvant est choisi parmi les éthers cycliques ou non cycliques, le diméthylformamide, le diméthylsulfoxyde, l'acétonitrile ou un solvant aromatique.

**7.** Procédé de préparation des composés de formule (II) par S-alkylation des 2-thiohydantoïnes de formule (III), en présence d'une base et dans un solvant, selon le schéma :

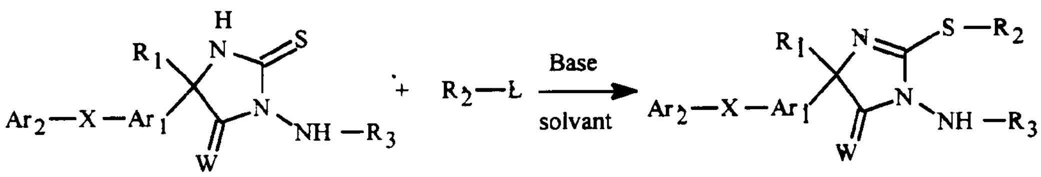


(III) (II)

dans lequel $R_1$, $R_2$, $R_3$, $Ar_1$, $Ar_2$, X sont définis selon la revendication 1, W représente S ou 0, et L représente un groupement choisi parmi un atome d'halogène, (de préférence chlore, brome, ou iode ), ou le groupe sulfate, alkylsulfonyloxy,ou arylsulfonyloxy.

**8.** Procédé de préparation des composés de formule (IIa), caractérisé en ce que l'on fait réagir successivement, en présence d'un solvant, le composé de formule (VII) avec une hydrazine de formule $R_3$-NH-$NH_2$, puis avec une base de formule $B^-M^+$, et enfin avec un agent d'alkylation de formule $R_2L$, selon le schéma:

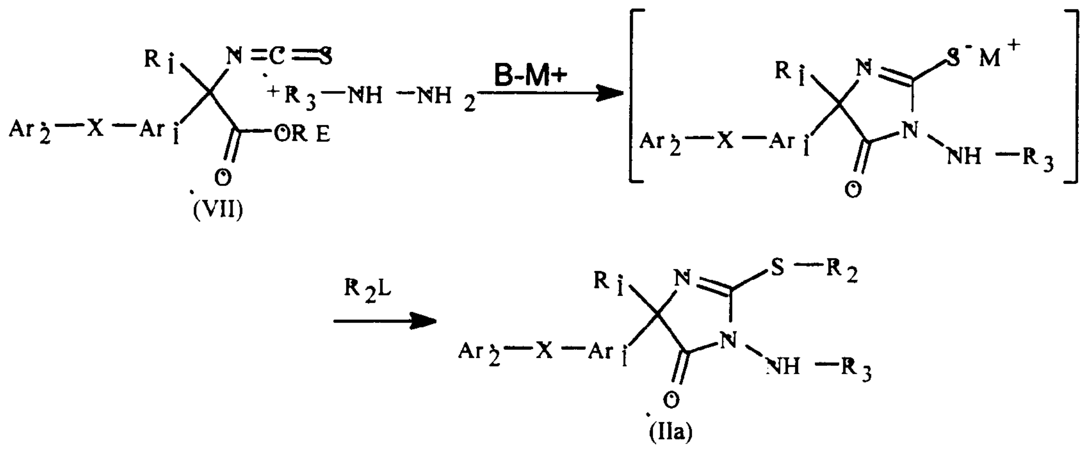


(VII)

(IIa)

dans lequel $R_1$, $R_2$, $R_3$, $Ar_1$, $Ar_2$, X sont définis selon la revendication 1, avec la restriction que $R_2$ est différent de l'atome d'hydrogène, $R_E$ représente un radical $C_1$-$C_3$ alkyle, et L est un atome d'halogène ou le groupe sulfate d'alkyle, alkylsulfonyloxy ou arylsulfonyloxy.

**9.** Procédé selon la revendication 8, caractérisé en ce que la base est choisie parmi un alcoolate alcalin ou alcalino-terreux, un hydroxyde alcalin ou alcalino-terreux, ou une amine tertiaire, et en ce que la réaction est réalisée à une température comprise entre -10 et +80°C et dans un solvant choisi notamment parmi un éther cyclique ou non cyclique, un alcool, un ester, le DMF, le DMSO, l'acétonitrile ou parmi un aromatique.

**10.** Procédé de préparation des composés de formule (XI), caractérisé en ce que l'on fait réagir dans un solvant les 2-alkylthio-2-imidazoline-5-ones de formule (II) avec un alcool $R_2OH$ en présence d'une base forte et dans un solvant, selon le schéma :

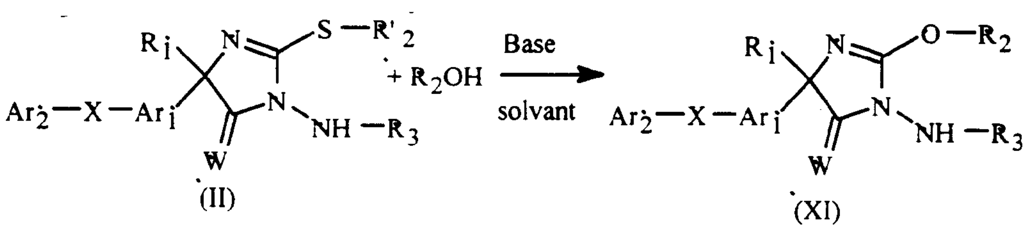


(II) (XI)

dans lequel $R'_2$ représente un groupe allyle comprenant de 1 à 3 atomes de carbone et les autres groupes sont définis selon la revendication 1.

**11.** Procédé selon la revendication 10, caractérisé en ce que la réaction est pratiquée dans l'alcool $R_2OH$, comme solvant, et en utilisant l'alcoolate de sodium $R_2O^-Na^+$ comme base, et en ce que la réaction est pratiquée à une température comprise entre 20 et 80°C.

**12.** Procédé de préparation des composés de formule (XII) :

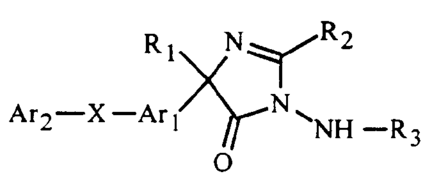

(XII)

caractérisé en ce que l'on fait réagir une hydrazine de formule $R_3$-NH-$NH_2$ sur une azalactone de formule (XIII), selon le schéma :

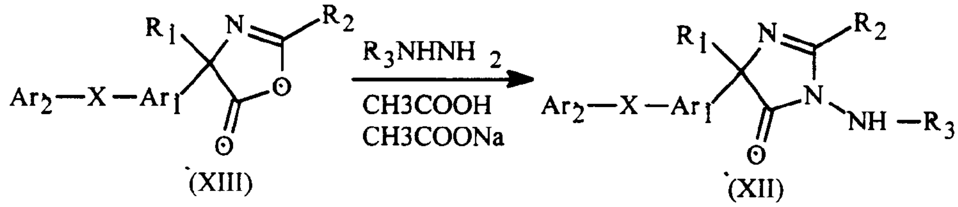


(XIII) (XII)

dans lequel $R_1$, $R_2$, $R_3$, $Ar_1$, $Ar_2$ et X sont définis selon la revendication 1, en chauffant le mélange des réactifs dans l'acide acétique au reflux, en présence d'acétate de sodium comme catalyseur.

**13.** Procédé de préparation des composés de formule (Ia) :

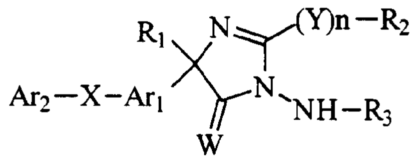

(Ia)

par condensation d'un dérivé organométallique de formule (XV) avec un radical électrophile $Ar_2^{-}X'^{+}$ selon le schéma :

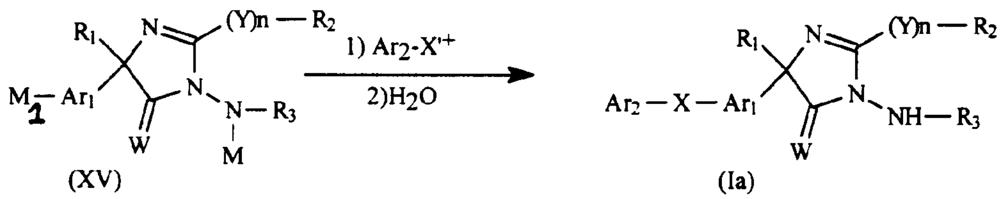


(XV) (Ia)

dans lequel :

* $R_1$, $R_2$, $R_3$, Y, $Ar_1$, $Ar_2$, W et n sont définis selon la revendication 1 ;
* $M_1$ représente un métal choisi parmi le lithium, le sodium, le magnésium, le zinc, le titane, l'étain, le cuivre, le bore ou le silicium et est de préférence le lithium ;
* $Ar_2$-X' est un radical électrophile généré par un composé choisi notamment parmi :
  - un halogénure ou un sulfate d'aralkyle de formule $Ar_2$-$R_{12}$-L
    dans laquelle :
    - $R_{12}$ représente une chaine aliphatique de 1 à 6 atomes de carbone, linéaire ou ramifiée, saturée ou insaturée éventuellement substituée par un halogène ou un groupe alkoxy ou un radical oxo ; et
    - L est défini selon la revendication 7 ;
  - un composé carbonylé de formule :

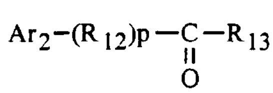

    dans laquelle :
    - p = 0 ou 1 ;
    - $R_{13}$ représente l'atome d'hydrogène, un groupe $C_1$-$C_6$ alkyle, un halogène, un groupe alkoxy, aryloxy, ou dialkylamino ;
  - un chloroformiate de formule :

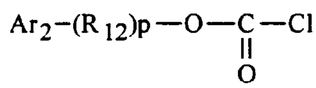

- un isocyanate ou un isothlocyanate de formule :

  $Ar_2$-$(R_{12})$p-N=C=Q

  dans laquelle Q représente l'atome d'oxygène ou de soufre ;
- un halogénure de sulfonyle de formule :

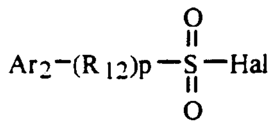

  dans laquelle Hal représente un atome d'halogène, de préférence l'atome de chlore ;
- un époxyde de formule :

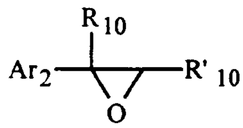

  dans laquelle $R_{10}$ et $R'_{10}$ sont définis selon la revendication 1 ;

* X a la même signification que X'.

**14.** Composés utiles notamment comme intermédiaires dans la préparation des composés selon la revendication 1, caractérisés en ce qu'ils répondent aux formules IIIa, IIIb, IV, VII, XIII, XIX, XX, XXII, XXIV et XXVII :

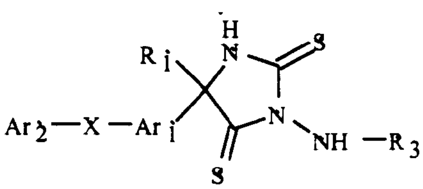

(IIIa)

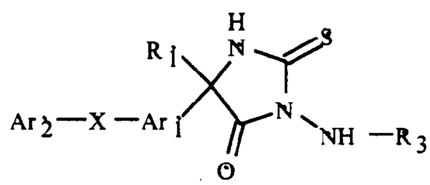

(IIIb)

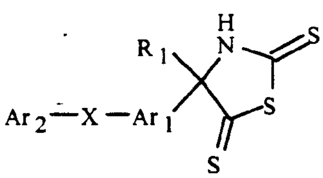

(IV)

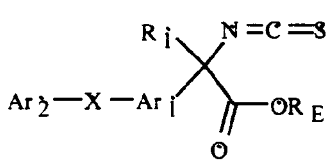

(VII)

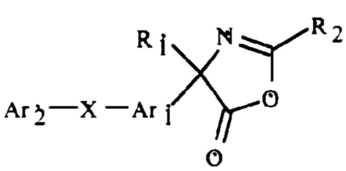

(XIII)

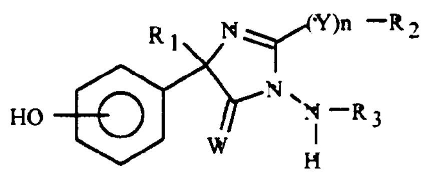

(XIX)

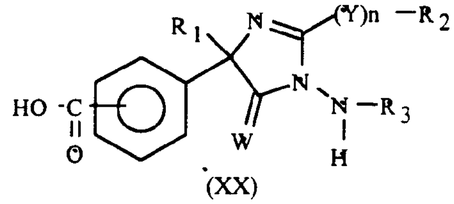

(XX)

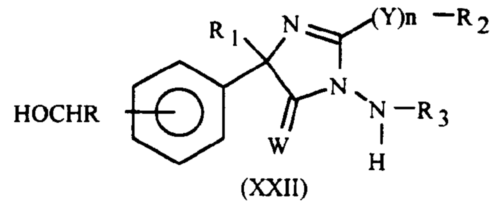

(XXII)

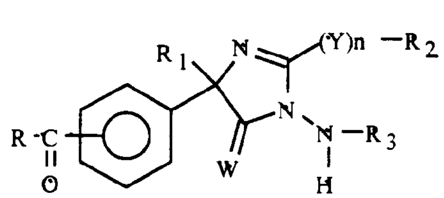

(XXIV)

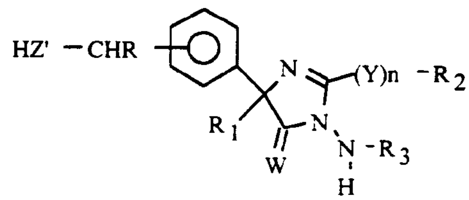

(XXVII)

dans lesquelles :

- $R_E$ est défini selon la revendication 8,
- Z' représente l'atome d'oxygène,de soufre, ou le groupe N-$R^{14}$, dans lequel $R_{14}$ représente l'atome d'hydrogène ou un groupe $C_1$-$C_6$ alkyle.
- les autres groupes sont définis selon la revendication 1 ;

ainsi que les isomères optiques de ces composés, notamment les énantiomères des composés comprenant le carbone asymétrique porteur de $R_1$.

**15.** Compositions fongicides, contenant comme matière(s) active(s) un (ou plusieurs) composé selon l'une des revendications 1 à 4, en mélange avec les supports solides ou liquides, acceptables en agriculture et les agents tensio-actifs également acceptables en agriculture.

**16.** Compositions fongicides selon la revendication 15, caractérisées en ce qu'elles contiennent de 0,05 à 95 % (en poids) de matières actives.

**17.** Procédé de traitement des plantes cultivées, atteintes ou susceptibles d'être atteintes par les maladies fongiques, caractérisé en ce que l'on applique sur les parties aériennes de ces plantes une dose efficace d'un composé selon l'une des revendications 1 à 4.

**18.** Procédé de traitement selon la revendication 17, caractérisé en ce que la dose efficace est comprise entre 0,002 et 5 kg/ha, de préférence entre 0,005 et 1 kg/ha.